(19) Europäisches Patentamt
European Patent Office
Office européen des brevets

(11) Veröffentlichungsnummer: **0 401 168 A2**

# (12) EUROPÄISCHE PATENTANMELDUNG

(21) Anmeldenummer: **90810380.7**

(22) Anmeldetag: **23.05.90**

(51) Int. Cl.5: **C07D 213/75, C07D 213/85, A01N 43/40, //C07D213/74**

(30) Priorität: **02.06.89 CH 2083/89**

(43) Veröffentlichungstag der Anmeldung: **05.12.90 Patentblatt 90/49**

(84) Benannte Vertragsstaaten: **AT BE CH DE DK ES FR GB GR IT LI LU NL SE**

(71) Anmelder: **CIBA-GEIGY AG**
**Klybeckstrasse 141**
**CH-4002 Basel(CH)**

(72) Erfinder: **Rempfler, Hermann, Dr.**
**Bruecklismattstrasse 16**
**CH-4107 Ettingen(CH)**

## (54) Neue Herbizide.

(57) Die Erfindung betrifft neue herbizide und pflanzenwuchsregulatorisch wirksame Mittel, enthaltend als Aktivsubstanz eine Verbindung der Formel I

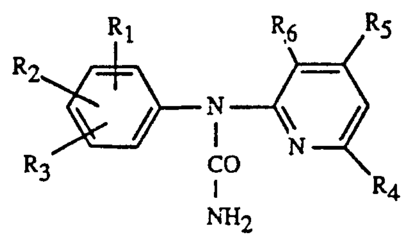

(I),

worin die Reste $R_1$ bis $R_6$ die in der Beschreibung gegebene Bedeutung haben, neue Verbindungen der Formel I und Zwischenprodukte und Verfahren zu deren Herstellung.

EP 0 401 168 A2

## Neue Herbizide

Die vorliegende Erfindung betrifft neue N-Phenyl-N-pyridin-2-yl-harnstoffe mit herbizider und pflanzenwuchsregulatorischer Wirkung, agrochemische Mittel, welche diese Substanzen als Wirkstoffe enthalten, die Verwendung der neuen Harnstoffe zur Bekämpfung von Unkräutern oder zur Regulierung des Pflanzenwuchses sowie Verfahren zur Herstellung der neuen Verbindungen. Ferner betrifft die Erfindung auch neue Zwischenprodukte und Verfahren zu deren Herstellung.

Aus US 2,802,008 sind im Pyridinteilsystem unsubstituierte N-Phenyl-N-pyridin-2-yl-und 3-yl-harnstoffe mit cardiovaskulärer Wirkung bekannt geworden. Demgegenüber wurde nun gefunden, dass bestimmte substituierte N-Phenyl-N-pyridin-2-yl-harnstoffe herbizid und pflanzenwuchsregulatorisch wirksam sind.

Die Erfindung betrifft herbizide und/oder wuchsregulatorische Mittel, enthaltend als Aktivsubstanz einen Harnstoff der Formel I

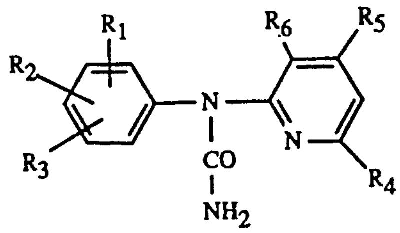

(I),

worin

$R_1$, $R_2$ und $R_3$ unabhängig voneinander Wasserstoff; Nitro; Cyano; Halogen; $C_1$-$C_4$-Alkyl; $C_1$-$C_4$-Alkyl-S(O)$_n$-; $C_1$-$C_4$-Alkoxy; $C_1$-$C_4$-Halogenalkyl; $C_1$-$C_4$-Halogenalkoxy; $C_1$-$C_4$-Halogenalkyl-S(O)$_n$-; $C_1$-$C_4$-Alkoxycarbonyl; $C_1$-$C_4$-Alkylcarbonyl; Aminocarbonyl; Mono-$C_1$-$C_4$-alkylaminnocarbonyl; oder Di-$C_1$-$C_4$-alkylaminocarbonyl;

$R_4$ und $R_5$ unabhängig voneinander Wasserstoff; $C_1$-$C_4$-Alkyl; $C_1$-$C_4$-Alkyl-S(O)$_n$-; $C_1$-$C_4$-Alkoxy; $C_1$-$C_4$-Halogenalkyl; $C_1$-$C_4$-Halogenalkoxy; $C_1$-$C_4$-Halogenalkyl-S(O)$_n$-; unsubstituiertes oder bis zu dreifach gleich oder verschieden durch $C_1$-$C_4$-Alkyl, Halogen, $C_1$-$C_4$-Alkoxy, $C_1$-$C_4$-Halogenalkyl, Nitro oder Cyano substituiertes Phenyl; Furanyl; Thiophenyl; $C_3$-$C_6$-Cycloalkyl; $C_1$-$C_4$-Alkoxycarbonyl; $C_1$-$C_4$-Alkoxy-$C_1$-$C_4$-alkyl; $C_1$-$C_4$-Alkoxycarbonyl-$C_1$-$C_4$-alkyl; $C_1$-$C_4$-Alkylcarbonyl-$C_1$-$C_4$-alkyl; $C_3$-$C_4$-Alkenyloxycarbonyl-$C_1$-$C_4$-alkyl; $C_3$-$C_4$-Alkinyloxycarbonyl-$C_1$-$C_4$-alkyl; Halogen; oder Cyano; und

$R_6$ Wasserstoff; $C_1$-$C_4$-Alkyl; Nitro; Cyano; Halogen; $C_1$-$C_4$-Alkoxycarbonyl; $C_1$-$C_4$-Halogenalkyl; und

n 0,1 oder 2

bedeutet unter Einschluss ihrer Salze mit Säuren, Basen und Komplexbildnern.

In den in dieser Beschreibung verwendeten Definitionen umfassen die angegebenen generischen Begriffe, sowie die durch Kombination einzelner Unterbegriffe erhältlichen Einzelbedeutungen der Substituenten, beispielsweise die folgenden Einzelsubstituenten, wobei diese Aufzählung keine Einschränkung der Erfindung darstellt.

Alkyl ist Methyl, Ethyl, n-Propyl, iso-Propyl, n-Butyl, sec.-Butyl, iso-Butyl, tert.-Butyl.

Halogen ist Fluor, Chlor, Brom und Jod. In erster Linie Fluor, Chlor und Brom.

Alkenyloxy und Alkinyloxy sind insbesondere Allyloxy und Propargyloxy. Diese ungesättigten Radikale können geradkettig oder verzweigt sein. Sie sind jeweils über ein gesättigtes Kohlenstoffatom an das Sauerstoffatom gebunden.

Cycloalkyl ist Cyclopropyl, Cyclobutyl, Cyclopentyl und Cyclohexyl, insbesondere Cyclopropyl, Cyclopentyl und Cyclohexyl.

Als Mono- oder Di-$C_1$-$C_4$-Alkylamino-Radikale sind insbesondere zu nennen: Methylamin, Dimethylamin, Methyl-ethylamin, Diisoropylamin, Monoisopropylamin.

Die Reste $C_1$-$C_4$-Alkyl-S(O)$_n$- umfassen sowohl die Thioether ($n = 0$) als auch die entsprechenden Sufinyl- und Sulfonyl-Reste (mit $n = 1$ oder 2). Bevorzugt sind Methylthio, Ethylthio, Methylsulfinyl, Ethylsulfinyl, Methylsulfonyl und Ethylsulfonyl.

Die Gruppe $C_1$-$C_4$-Halogenalkyl-S(O)$_n$- steht für die jeweiligen Halogenalkylthio-, Halogenalkylsulfinyl- und Halogenalkylsulfonylradlkale. Besonders bevorzugt sind Difluormethylthio, Difluormethylsulfinyl, Difluormethylsulfonyl, Trifluormethylthio oder Chlorfluormethylthio.

Mit Halogenalkyl sind die ganz oder teilweise gleich oder verschieden halogensubstituierten Alkyle

gemäss der jeweiligen Delinitionsbreite gemeint, wie etwa Trifluormethyl, Difluormethyl, 1,1,2,2-Tetrafluorethyl, 2-Chlorethyl, Pentafluorethyl, Chlordifluormethyl, Dichlormethyl, Chlorfluormethyl, 1,1-Dichlor-2,2,2-trifiuorethyl, 1,1-Dichlorethyl, Brommethyl oder Heptafluorethyl.

Als $C_1$-$C_4$-Alkoxycarbonylreste sind unter anderem Methoxycarbonyl, Ethoxycarbonyl sowie die isomeren Propyloxycarbonyle und Butyloxycarbonyle zu nennen.

Alkoxy ist Methoxy, Ethoxy, i-Propyloxy, n-Propyloxy, n-Butyloxy, t-Butyloxy, i-Butyloxy und sec-Butyloxy.

Das als Substituent genannte Phenylradikal kann innerhalb der angegebenen Definitionsbreite mit gleichen oder verschiedenen Substituenten substituiert sein. Bevorzugt ist das Phenylradikal unsubstituiert oder bis zu dreifach substituiert. Als Einzelbedeutungen sind neben dem unsubsituierten Phenyl, 2-Chlorphenyl, 4-Chlorphenyl, 2,4-Dichlorphenyl, 2-Methylphenyl, 4-Methylphenyl, 2-Trifluormethylphenyl und 4-Trifluormethylphenyl zu nennen.

Halogenalkoxy sind im Rahmen der jeweiligen Delinitionsbreite die isomeren ein- oder mehrfach gleich oder verschieden halogensubstituierten Reste, wie etwa Trifluormethoxy, 2,2,2-Trifluorethoxy, 2,2,3,3,3-Pentafluorpropyloxy, 1,1,2,2-Tetrafluorethoxy, Difluormethoxy oder 2-Chlorethoxy.

Als Alkoxyalkylreste sind unter anderem zu nennen: 2-Ethoxyethyl, 2-Methoxyethyl, 3-Methoxypropyl, 2-Methoxy-1-methylethyl und Methoxymethyl.

Bedingt durch ihre chemische Konstitution können die Verbindungen der Formel I zahlreiche Salze mit Säuren und Basen bilden. Die Erfindung umfasst auch diese Salze mit agrochemisch verträglichen Säuren und Basen. Das gleiche gilt für Komplexe und Komplexbildner.

Hervorzuheben sind Mittel, welche als Aktivsubstanz eine in der 4-Position des Phenylringes unsubstituierte Verbindunge der Formel Ia

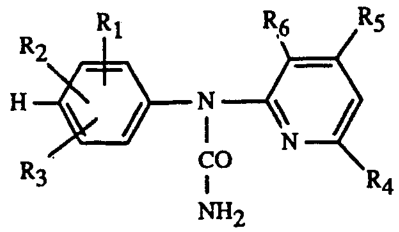

(Ia),

worin die Reste $R_1$ bis $R_6$ wie zuvor definiert sind, enthalten.

Insbesondere betrifft die Erfindung Mittel, welche als Aktivsubstanz eine Verbindung der Formel Ia

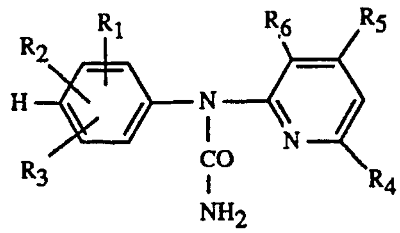

(Ia),

worin $R_1$, $R_2$ und $R_3$ unabhängig voneinander Wasserstoff; nitro; Halogen; $C_1$-$C_4$-Alkyl; $C_1$-$C_4$-Halogenalkyl; $C_1$-$C_4$-Alkoxy; oder $C_1$-$C_4$-Halogenalkoxy;

$R_4$ und $R_5$ unabhängig voneinander Wasserstoff; Cyano; Halogen; $C_1$-$C_4$-Alkyl; $C_1$-$C_4$-Alkoxy; $C_1$-$C_4$-Halogenalkyl; $C_1$-$C_4$-Alkoxy-$C_1$-$C_4$-alkyl; Phenyl; oder Furanyl; und

$R_6$ Wasserstoff; Cyano; Nitro; Halogen; oder $C_1$-$C_4$-Alkoxycarbonyl;

bedeutet, enthalten.

Bevorzugt sind Mittel, welche als Aktivsubstanz eine Verbindung der Formel Ia

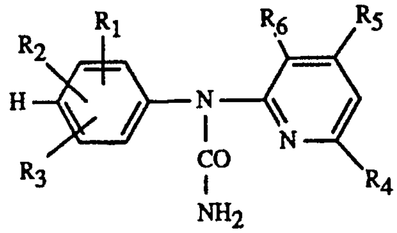

(Ia),

worin
$R_1$ Wasserstoff; Nitro; Halogen; $C_1$-$C_4$-Halogenalkyl; oder $C_1$-$C_4$-Halogenalkoxy;
$R_2$ Wasserstoff; Halogen; oder $C_1$-$C_4$-Alkyl;
$R_3$ Wasserstoff; $C_1$-$C_4$-Alkyl; oder Halogen;
$R_4$ Cyano; Halogen; $C_1$-$C_4$-Alkyl; $C_1$-$C_4$-Alkoxy; $C_1$-$C_4$-Halogenalkyl; $C_1$-$C_4$-Alkoxy-$C_1$-$C_4$-alkyl; Phenyl; oder Furanyl;
$R_5$ Halogen; $C_1$-$C_4$-Alkyl; $C_1$-$C_4$-Halogenalkyl; oder Phenyl; und
$R_6$ Wasserstoff; Cyano; Nitro; Halogen; oder $C_1$-$C_4$-Alkoxycarbonyl
bedeutet, enthalten.

Hervorzuheben sind die Mittel, welche als Aktivsubstanz eine Verbindung der Formel Ia,

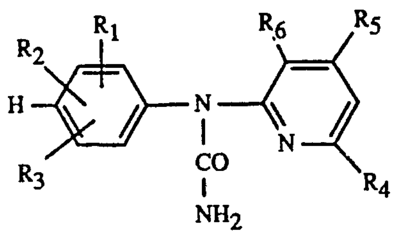

(Ia),

worin
$R_1$ Wasserstoff; Fluor; Chlor; Brom; Jod; Nitro; Trifluormethyl; Methoxy; Trifluormethoxy; oder Difluormethoxy;
$R_2$ Wasserstoff; Fluor; Chlor; oder Methyl;
$R_3$ Wasserstoff; Chlor; oder Methyl;
$R_4$ Chlor; Brom; $C_1$-$C_4$-Alkyl; Cyano; Methoxy; Trifluormethyl; Methoxymethyl; Phenyl; oder Furanyl;
$R_5$ Chlor; Methyl; Trifluormethyl; Chlordifluormethyl; Difluormethyl; Dichlormethyl; oder Pentafluorethyl; und
$R_6$ Wasserstoff; Cyano; Nitro; Chlor; Brom; oder Methoxycarbonyl;
bedeutet, enthalten.

Von den vorstehend genannten Mitteln, welche als Aktivsubstanz eine Verbindung der Formel I enthalten, und den hervorgehobenen bzw. bevorzugt genannten Verbindungen der Formel Ia sind nachstehende Isomere der Formeln $Ia^1$ bis $Ia^6$ besonders herausgestellt:

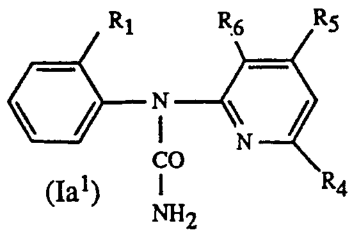
(Ia¹)

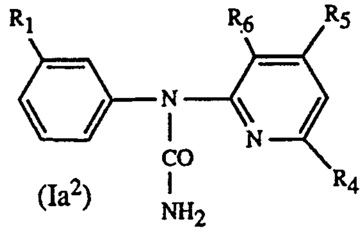
(Ia²)

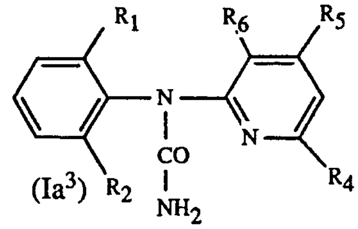
(Ia³)

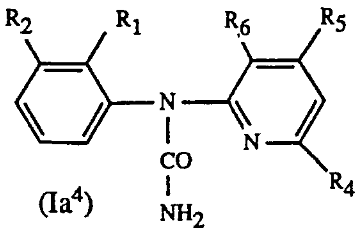
(Ia⁴)

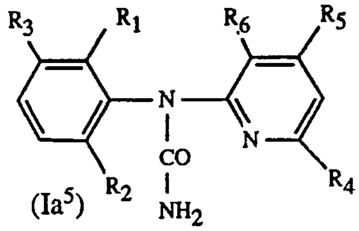
(Ia⁵)

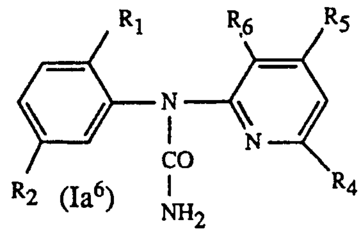
(Ia⁶)

Hervorzuheben sind insbesondere auch Mittel, welche als Aktivsubstanz eine Verbindung der Formel Ia, enthalten, worin die Reste

$R_1$, $R_2$ und $R_3$ wie zuvor definiert sind;

$R_4$ und $R_5$ unabhängig voneinander jeweils Halogen-$C_1$-$C_4$-alkyl; oder $C_1$-$C_4$-Alkyl; und

$R_6$ Wasserstoff;

bedeutet.

Weiterhin hervorzuheben sind auch Mittel, welche als Aktivsubstanz Verbindungen der Formel $Ia^1$ oder $Ia^3$, worin die Reste

$R_1$ und $R_2$ wie zuvor definiert sind;

$R_6$ Wasserstoff; und

$R_4$ und $R_5$ unabhängig voneinander je Halogen-$C_1$-$C_4$-alkyl, insbesondere Trifluormethyl; oder $C_1$-$C_4$-Alkyl; bedeutet, enthalten.

Ebenfalls hervorzuheben sind Mittel, welche als Aktivsubstanz Verbindungen der Formel $Ia^1$ oder $Ia^3$, worin

$R_1$ Nitro;

$R_2$ wie zuvor definiert ist; und

$R_4$ und $R_5$unabhängig voneinander je Halogen-$C_1$-$C_4$-alkyl, insbesondere Trifluormethyl; oder $C_1$-$C_4$-Alkyl; bedeutet, enthalten.

Ein weiterer Erfindungsgegenstand sind die neuen Harnstoffe der Formel I

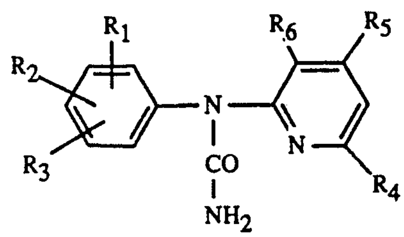
(I),

worin

$R_1$, $R_2$ und $R_3$ unabhängig voneinander Wasserstoff; Nitro; Cyano; Halogen; $C_1$-$C_4$-Alkyl; $C_1$-$C_4$-Alkyl-$S(O)_n$-; $C_1$-$C_4$-Alkoxy; $C_1$-$C_4$-Halogenalkyl; $C_1$-$C_4$-Halogenalkoxy; $C_1$-$C_4$-Halogenaikyl-$S(O)_n$-; $C_1$-$C_4$-Alkoxycarbonyl; $C_1$-$C_4$-Alkylcarbonyl; Aminocarbonyl; Mono-$C_1$-$C_4$-alkylaminnocarbonyl; oder Di-$C_1$-$C_4$-alkylaminocarbonyl;

$R_4$ und $R_5$ unabhängig voneinander Wasserstoff; $C_1$-$C_4$-Alkyl; $C_1$-$C_4$-Alkyl-$S(O)_n$-; $C_1$-$C_4$-Alkoxy; $C_1$-$C_4$-Halogenalkyl; $C_1$-$C_4$-Halogenalkoxy; $C_1$-$C_4$-Halogenalkyl-$S(O)_n$-; unsubstituiertes oder bis zu dreifach gleich oder verschieden durch $C_1$-$C_4$-Alkyl, Halogen, $C_1$-$C_4$-Alkoxy, $C_1$-$C_4$-Halogenalkyl, Nitro oder Cyano substituiertes Phenyl; Furanyl; Thiophenyl; $C_3$-$C_6$-Cycloalkyl; $C_1$-$C_4$-Alkoxycarbonyl; $C_1$-$C_4$-Alkoxy-$C_1$-$C_4$-alkyl;

$C_1$-$C_4$-Alkoxycarbonyl-$C_1$-$C_4$-alkyl; $C_1$-$C_4$-Alkylcarbonyl-$C_1$-$C_4$-alkyl; $C_3$-$C_4$-Alkenyloxycarbonyl-$C_1$-$C_4$-alkyl; $C_3$-$C_4$-Alkinyloxycarbonyl-$C_1$-$C_4$-alkyl; Halogen; oder Cyano; und
$R_6$ Wasserstoff; $C_1$-$C_4$-Alkyl; Nitro; Cyano; Halogen; $C_1$-$C_4$-Alkoxycarbonyl; $C_1$-$C_4$-Halogenalkyl; und
n 0,1 oder 2
bedeutet, mit der Massgabe, dass die Reste $R_4$, $R_5$ und $R_6$ nicht alle zugleich für Wasserstoff stehen könnnen, unter Einschluss ihrer Salze mit Säuren, Basen und Komplexbildnem.
Hervorzuheben sind die in der 4-Position des Phenylringes unsubstituierten Verbindungen der Formel Ia

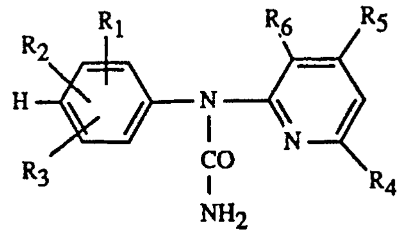

(Ia),

worin die Reste $R_1$ bis $R_6$ wie zuvor definiert sind.

Insbesondere betrifft die Erfindung Verbindungen der Formel Ia

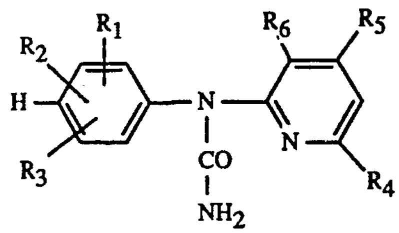

(Ia),

worin
$R_1$, $R_2$ und $R_3$ unabhängig voneinander Wasserstoff; Nitro; Halogen; $C_1$-$C_4$-Alkyl; $C_1$-$C_4$-Halogenalkyl; $C_1$-$C_4$-Alkoxy; oder $C_1$-$C_4$-Halogenalkoxy;
$R_4$ und $R_5$ unabhängig voneinander Wasserstoff; Cyano; Halogen; $C_1$-$C_4$-Alkyl; $C_1$-$C_4$-Alkoxy; $C_1$-$C_4$-Halogenalkyl; $C_1$-$C_4$-Alkoxy-$C_1$-$C_4$-alkyl; Phenyl; oder Furanyl; und
$R_6$ Wasserstoff; Cyano; Nitro; Halogen; oder $C_1$-$C_4$-Alkoxycarbonyl;
bedeutet.

Bevorzugt sind die Verbindungen der Formel Ia

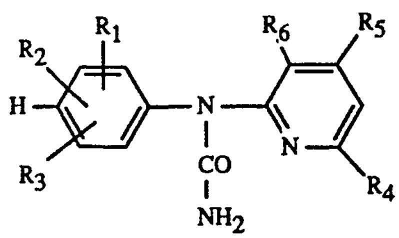

(Ia),

worin
$R_1$ Wasserstoff; Nitro; Halogen; $C_1$-$C_4$-Halogenalkyl; oder $C_1$-$C_4$-Halogenalkoxy;
$R_2$ Wasserstoff; Halogen; oder $C_1$-$C_4$-Alkyl;
$R_3$ Wasserstoff; $C_1$-$C_4$-Alkyl; oder Halogen;
$R_4$ Cyano; Halogen; $C_1$-$C_4$-Alkyl; $C_1$-$C_4$-Alkoxy; $C_1$-$C_4$-Halogenalkyl; $C_1$-$C_4$-Alkoxy-$C_1$-$C_4$-alkyl; Phenyl; oder Furanyl;
$R_5$ Halogen; $C_1$-$C_4$-Alkyl; $C_1$-$C_4$-Halogenalkyl; oder Phenyl; und
$R_6$ Wasserstoff; Cyano; Nitro; Halogen; oder $C_1$-$C_4$-Alkoxycarbonyl
bedeutet.

Hervorzuheben sind die Harnstoffe der Formel Ia,

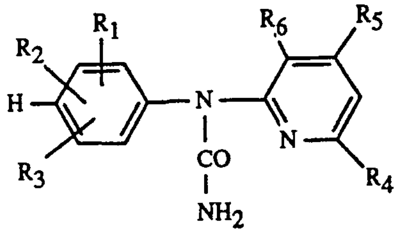

(Ia),

worin

$R_1$ Wasserstoff; Fluor; Chlor; Brom; Jod; Nitro; Trifluormethyl; Methoxy; Trifluormethoxy; oder Difluormethoxy;

$R_2$ Wasserstoff; Fluor; Chlor; oder Methyl;

$R_3$ Wasserstoff; Chlor; oder Methyl;

$R_4$ Chlor; Brom; $C_1$-$C_4$-Alkyl; Cyano; Methoxy; Trifluormethyl; Methoxymethyl; Phenyl; oder Furanyl;

$R_5$ Chlor; Methyl; Trifluormethyl; Chlordifluormethyl; Difluormethyl; Dichlormethyl; oder Pentafluorethyl; und

$R_6$ Wasserstoff; Cyano; Nitro; Chlor; Brom; oder Methoxycarbonyl;bedeutet.

Von den vorstehend genannten Verbindungen der Formel I und den hervorgehobenen bzw. bevorzugt genannten Verbindungen der Formel Ia sind nachstehenden Isomere der Formeln $Ia^1$ bis $Ia^6$ besonders herausgestellt:

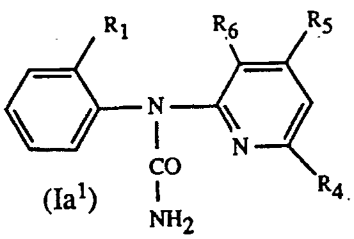

($Ia^1$)

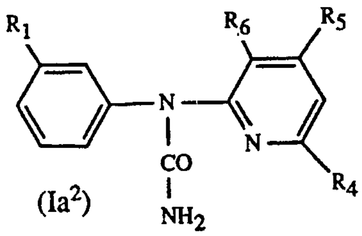

($Ia^2$)

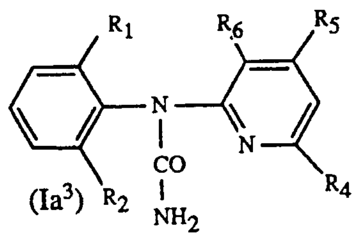

($Ia^3$)

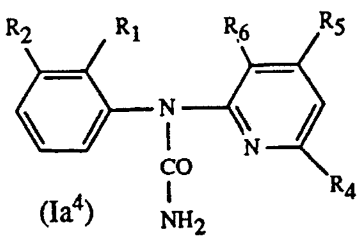

($Ia^4$)

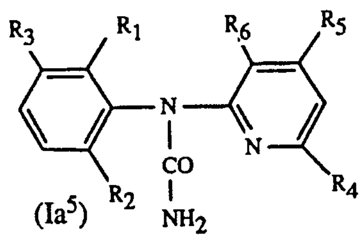

($Ia^5$)

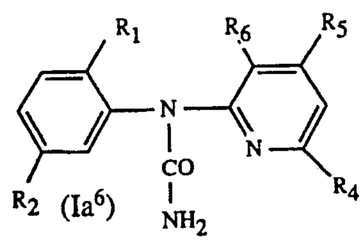

($Ia^6$)

Hervorzuheben sind insbesondere auch Verbindungen der Formel Ia, worin die Reste $R_1$, $R_2$ und $R_3$ wie zuvor definiert sind;

$R_4$ und $R_5$ unabhängig voneinander jeweils Halogen-$C_1$-$C_4$-alkyl, insbesondere Trifluormethyl; oder $C_1$-$C_4$-Alkyl; und

$R_6$ Wasserstoff;

bedeutet.

Weiterhin hervorzuheben sind die Verbindungen der Formel $Ia^1$ oder $Ia^3$, worin die Reste

$R_1$ und $R_2$ wie zuvor definiert sind;

$R_6$ Wasserstoff; und

$R_4$ und $R_5$ unabhängig voneinander je Halogen-$C_1$-$C_4$-alkyl, insbesondere Trifluormethyl; oder $C_1$-$C_4$-Alkyl; bedeutet.

Ebenfalls hervorzuheben sind die Verbindungen der Formel $Ia^1$ oder $Ia^3$, worin

$R_1$ Nitro;

$R_2$ wie zuvor definiert ist ; und

$R_4$ und $R_5$unabhängig voneinander je Halogen-$C_1$-$C_4$-alkyl, insbesondere Trifluormethyl; oder $C_1$-$C_4$-Alkyl; bedeutet.

Die Verbindungen der Formel I können hergestellt werden, dadurch, dass man

a) ein Anilin der Formel II, worin die Reste $R_1$ bis $R_6$ wie unter Formel I definiert sind, mit Phosgen zu einem Carbaminchlorid der Formel III umsetzt und dieses in einer zweiten Stufe mit $NH_3$ zu einem Harnstoff der Formel I umsetzt

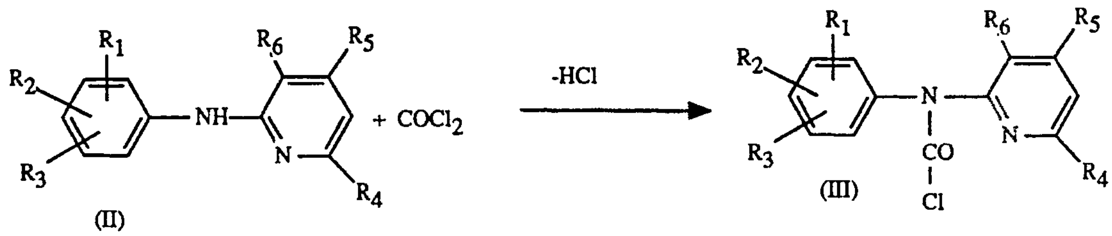


(II) (III)

$$III + NH_3 \xrightarrow{-HCl} I$$

oder

b) ein Anilin der Formel II, worin die Reste $R_1$ bis $R_6$ wie unter Formel I definiert sind, mit Halogensulfonylisocyanat V zu einem Halogensulfonylharnstoff der Formel IV umsetzt und diesen dann in einer zweiten Stufe oder direkt zu einer Verbindung der Formell hydrolisiert, wobei Y für eine unter den Reaktionsbedingungen abspaltbare Gruppe, wie Halogen, vorzugsweise Chlor, steht:

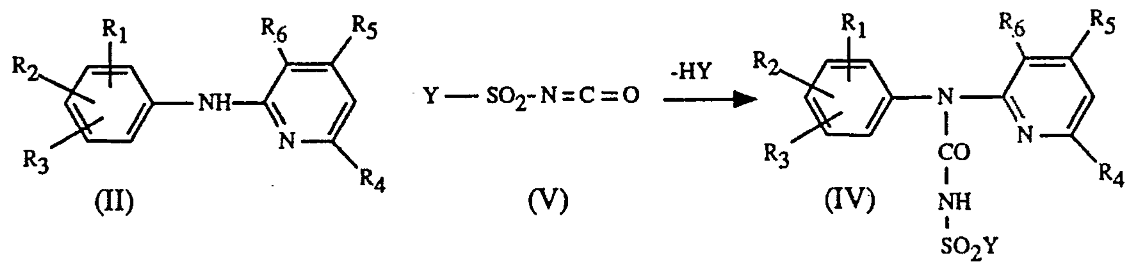


(II) (V) (IV)

$$IV + H_2O \xrightarrow[-SO_4H_2]{-HY} I$$

Weiterhin können Harnstoffe der Formel I′

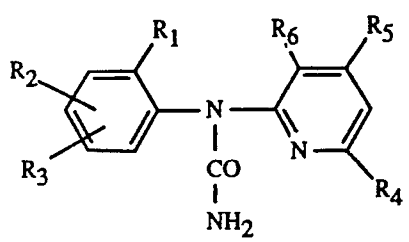

(I‘), ($R^1 = NO_2$)

worin $R^1$ in der ortho-Stellung des Phenylringes gebunden ist und Nitro bedeutet und die Reste $R_2$ bis $R_6$ wie unter Formel I definiert sind, hergestellt werden, dadurch dass man

c) einen Sulfonylharnstoff der Formel VI unter Einwirkung einer wässrigen Base zu einem Harnstoff der Formel I′ umlagert, wobei als wässrige Basen vorzugsweise NaOH/Wasser oder KOH/Wasser verwendet werden

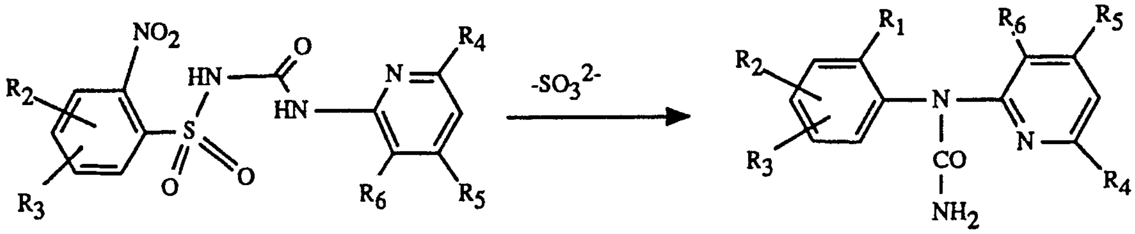


(VI) (I') ($R^1 = NO_2$)

Die unter Halogenwasserstoffabspaltung bzw HY-Eliminierung verlaufenden Umsetzungen II → III,II → IV und III → I werden vorzugsweise unter Verwendung säurebindender Mittel (Basen) durchgeführt.

Als solche kommen organische oder anorganische Basen in Betracht, z.B. tertiäre Amine, wie Trialkylamine (Trimethylamin, Triethylamin, Tripropylamin usw.), Pyridine (Pyridin, 4-Dimethylaminopmdin, 4-Pyrtolidinylaminopmdin usw.), Alkoholate, wie z.B. Kalium-tert.-butylat, Natrium-methanolat, Natrium-ethanolat etc.. Die unter Baseneinwirkung verlaufenden Reaktionen, wie auch die Umsetzung VI → I', können auch unter Phasentransferbedingungen mit Basen nach an sich bekannten Verfahren durchgeführt werden (Lit. Dehmlow & Dehmlow, Phase Transfer Catalysis; Verlag Chemie, Weinheim, 1983).

Ein weiterer Erfindungsgegenstand sind die neuen Verbindungen der Formel II

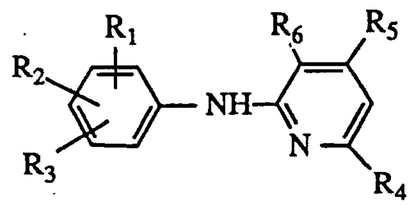
(II)

worin die Reste

$R_1$ bis $R_6$ wie unter Formel I definiert sind.

Besonders bevorzugt sind die Aniline der Formel $IIa^1$ oder $IIa^3$

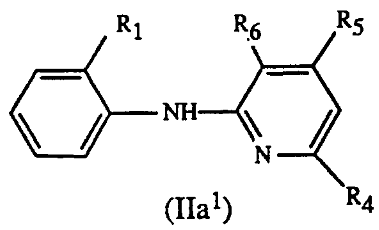
($IIa^1$)

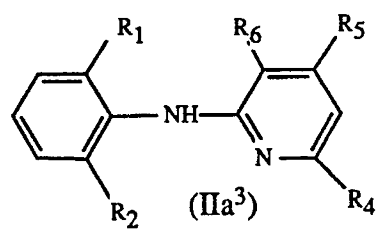
($IIa^3$)

Weiterhin hervorzuheben sind die Verbindungen der Formel $IIa^1$ oder $IIa^3$, worin die Reste

$R_1$ und $R_2$ wie zuvor definiert sind;

$R_6$ Wasserstoff; und

$R_4$ und $R_5$ unabhängig voneinander je Halogen-$C_1$-$C_4$-alkyl, insbesondere Trifluormethyl; oder $C_1$-$C_4$-Alkyl; bedeutet.

Ebenfalls hervorzuheben sind die Verbindungen der Formel $IIa^1$ oder $IIa^3$, worin

$R_1$ Nitro;

$R_2$ wie zuvor definiert ist;

$R_4$ und $R_5$ unabhängig voneinander je Halogen-$C_1$-$C_4$-alkyl, insbesondere Trifluormethyl; oder $C_1$-$C_4$-Alkyl; und

$R_6$ Wasserstoff;

bedeutet.

Die Verbindungen der Formel II können hergestellt werden, dadurch dass man

a) ein Anilin der Formel $VII_1$, worin die Reste $R_1$ bis $R_3$ wie zuvor definiert sind, mit einem Pyridin der

Formel III, worin die Reste $R_4$ bis $R_6$ wie unter Formel II definiert sind und X Halogen, $C_1$-$C_4$-Alkyl-$SO_2$-, oder Phenyl-$SO_2$- bedeutet, unter Baseneinwirkung umsetzt

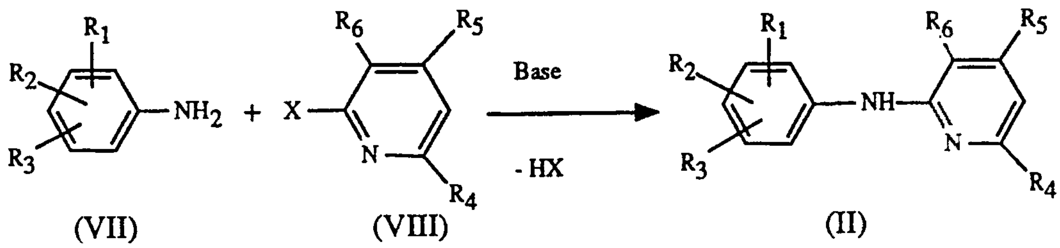


(VII) (VIII) (II)

oder

b) ein Halogenbezol der Formel IX, worin die Reste $R_1$, $R_2$ und $R_3$ wie unter Formel II definiert sind und Y Halogen bedeutet, unter Baseneinwirkung mit einem 2-Aminopyridin der Formel X umsetzt

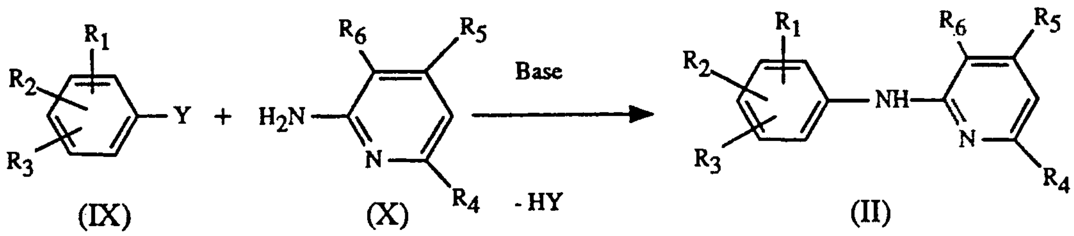


(IX) (X) (II)

Als Basen für die vorstehend angegebenen Verfahren zur Herstellung von Verbindungen der Formel II sind Alkalimetallhydride, wie Natriumhydrid oder Kaliumhydrid, Alkylimetallamide, wie Natriumamid oder Kaliumamid oder Metallorganische Verbindungen, wie Butyllithium oder Phenyllithium zu nennen.

Weiterhin betrifft die Erfindung die neuen Carbaminchloride der Formel III

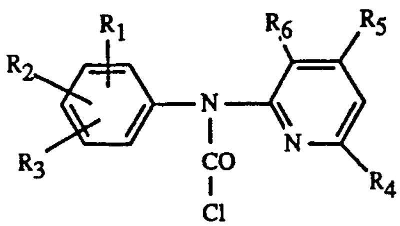
(III),

worin die Reste $R_1$ bis $R_6$ wie unter Formel I definiert sind.

Die Verbindungen der Fomel III können hergestellt werden, dadurch dass man ein Anilin der Formel II, worin die Reste $R_1$ bis $R_6$ wie unter Formel I definiert sind mit Phosgen umsetzt

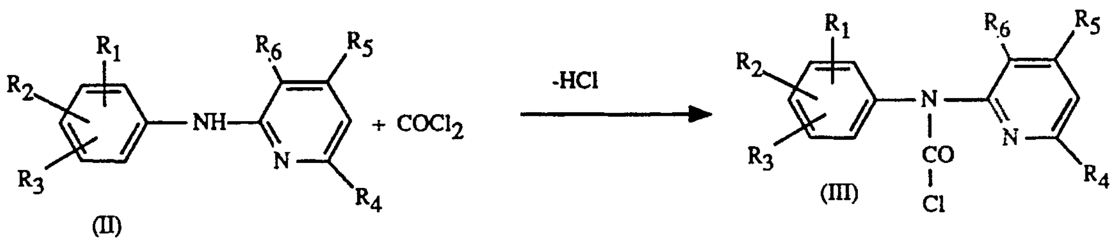


(II) (III)

Die Erfindung betrifft auch die neuen Halogensulfonylharnstoffe der Formel IV

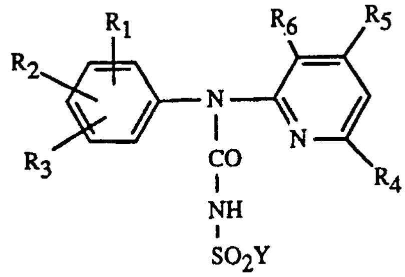

(IV),

worin die Reste $R_1$ bis $R_6$ wie unter Formel I definiert sind.

Die Verbindungen der Formel IV können hergestellt werden, dadurch dass man ein Anilin der Formel II, worin die Reste $R_1$ bis $R_6$ wie unter Formel I definiert sind, mit einem Halogensulfonylisocyanat der Formel V, worin Y für Halogen, insbesondere Chlor steht, umsetzt

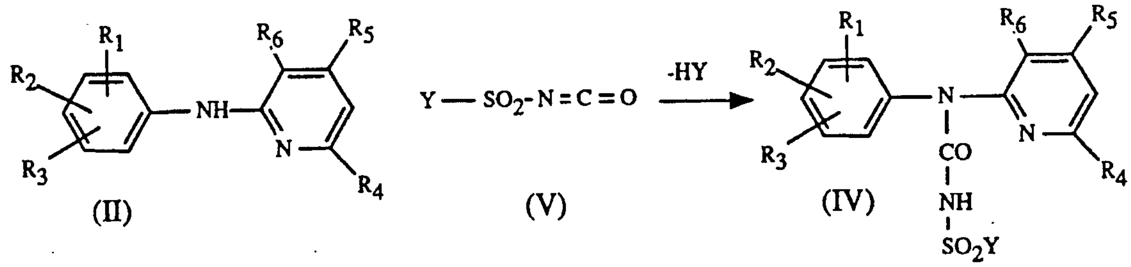


(II) (V) (IV)

Die Verbindungen der Formel I sind hochaktive Herbizide, die sich bei entsprechenden Aufwandmengen in vorteilhafter Weise auch als Selektivherbizide zur Unkrautbekämpfung in Nutzpflanzenkulturen eignen, Kulturpflanzen wie Getreide (wie Roggen, Gerste, Hafer, Weizen), Mais, Hirse, Reis, Baumwolle, Soja, Raps und Sonnenblumen bleiben bei niedrigen Aufwandmengen praktisch ungeschädigt. Bei gesteigerten Aufwandmengen werden die Kulturpflanzen nur geringfügig in ihrem Wachstum beeinflusst. Werden sehr hohe Aufwandmengen appliziert, entfalten die Substanzen der Formel I totalherbizide Eigenschaften.

Die selektiv-herbizide Wirkung der erfindungsgemässen Verbindungen wird sowohl bei der preemergenten als auch der postemergenten Anwendung festgestellt. Diese Wirkstoffe können daher im Vorauflaufverfahren und im Nachauflaufverfahren zur selektiven Unkrautbekämpfung mit gutem Erfolg verwendet werden. Bevorzugt werden die Verbindungen der Formel I aber im Vorauflauf eingesetzt.

Die Erfindung betrifft auch herbizide Mittel, welche einen neuen Wirkstoff der Formel I enthalten, sowie Verfahren zur pre- und postemergenten Unkrautbekämpfung.

Die Verbindungen der Formel I haben ausserdem pflanzenwuchsregulatorische Eigenschaften. Es werden sowohl Monokotyledonen als auch Dikotyledonen in ihrem Wachstum beeinflusst.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertagssteigerung mit Wachstumsregulatoren beruht darauf, dass die Nährstoffe in stärkerem Masse der Blüten- und Fruchtbildung zugute kommen, während das vegetative Wachstum eingeschränkt wird.Bei grösseren Aufwandmengen werden Unkräuter und Gräser in ihrer Entwicklung so geschädigt, dass sie absterben.

In besonders vorteilhafter Weise können die wuchsregulatorischen Verbindungen der Formel I zur Wuchsregulation von Untersaaten in Maiskulturen verwendet werden.

Als Untersaaten in Maiskulturen sind prinzipiell diejenigen Pflanzen geeignet, die den Boden zwischen den einzelnen Maispflanzen bedecken und so in erster Linie der Bodenerosion in Maiskulturen entgegenwirken. Geeignete Pflanzen für die Untersaat sind unter anderem Raps, Klee, Gräser oder Leguminosen.

In geeigneten Aufwandmengen hemmen die Verbindungen der Formel I den Neuzuwachs von Gräsern. Dies erlaubt es in Rasenkulturen (Parks, Gärten etc.) die Zahl der erforderlichen Schnitte zu vermindern, beziehungsweise die Zeiträume zwischen den einzelnen Schnitten zu verlängern. In besonders vorteilhafter Weise können hierzu Granulatformulierungen der Wirkstoffe der Formel I verwendet werden. Das Granulat kann entweder den Wirkstoff allein, neben den üblichen Hilfs- und Trägerstoffen, enthalten oder der Wirkstoff wird zusammen mit einem Mineraldünger, und/oder gegebenenfalls mit weiteren Wirkstoffen zur Bekämpfung von Moos und anderem in Rasenkulturen unerwünschten Pflanzenwuchses, als Granulat formuliert. Die Anwendung als Streugranulat erlaubt es, mit Hilfe der bei Rasenkulturen üblichen Bearbeitungsgeräte das Granulat auszubringen und so während längerer Zeit den Neuzuwachs der Gräser zu hemmen. Das Granulat kann dabei in an sich bekannter Weise hergestellt werden, es weist vorzugsweise eine Korngrösse von 0,1 bis 2,0 mm, insbesondere von 0,25 bis 1,0 mm, auf.

Die Wirkstoffe der Formel I werden in der Regel mit Aufwandmengen von 0,005 bis 5 kg/ha insbesondere 0,1 bis 3 kg/ha erfolgreich eingesetzt. Die für die erwünschte Wirkung erforderliche Dosierung kann durch Versuche ermittelt werden. Sie ist abhängig von der Art der Wirkung (Selektivherbizid, Totalherbizid, Wuchsregulation), dem Entwicklungsstadium der Kulturpflanze und des Unkrauts sowie der Applikation (Ort, Zeit, Applikationsverfahren) und kann, bedingt durch diese Parameter, innerhalb weiter Bereiche variieren.

In vorteilhafter Weise können die erfindungsgemässen Wirkstoffe auch auf das Vermehrungsgut der Kuturpflanze aufgebracht werden. Vermehrungsgut sind Samen, Stecklinge oder sonstige Teile der Pflanze, aus denen die Kulturpflanze gezogen werden kann. Besonders zu erwähnen ist hier die Samenbeizung. Das mit einer wirksamen Menge einer Verbindung der Formel I behandelte Vermehrungsgut ist ebenfalls Gegenstand vorliegender Erfindung.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

So können die Aktivsubstanzen der Formel I auch auf mineralische Dünger aufgebracht (aufgebeizt) werden.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kationen- und/oder anionenaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearin- säure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nicht ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 10 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethoxy ethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedere, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ethylammoniumbromid.

Klebstoffe sind insbesondere diejenigen Hilfsstoffe, welche beim Granulieren den Zusammenhalt des Trägermaterials, der Hilfsstoffe und der Wirkstoffe bewirken, wie Gummi arabicum oder Carboxymethylcellulose

Die in der Formulierungstechnik gebräuchlichen Tenside sind u.a. in folgenden Publikationen beschrieben:

"1987 International Mc Cutcheon's Emulsifiers and Detergents", Glen Rock, N.J., USA.

Dr. Helmut Stache "Tensid Taschenbuch" Carl Hanser Verlag, München/Wien 1981.

Die Zubereitungen enthalten in der Regel 0,1 bis 95 %, insbesondere 0,1 bis 80 %, Wirkstoff der Formel I, 1 bis 99,9 % eines festen oder flüssigen Zusatzstoffs und 0 bis 25 %, insbesondere 0,1 bis 25 % eines Tensides.

Insbesondere setzen sich bevorzugte Formulierungen folgendermassen zusammen: (% = Gewichtsprozent)

| Emulgierbare Konzentrate: | |
|---|---|
| Wirkstoff der Formel I:<br>oberflächenaktives Mittel:<br>flüssiges Trägermittel: | 1 bis 20 %, bevorzugt 5 bis 10 %<br>5 bis 30 %, vorzugsweise 10 bis 20 %<br>50 bis 94 %, vorzugsweise 70 bis 85 %. |
| Stäube: | |
| Wirkstoff der Formel I:<br>festes Trägermittel: | 0,1 bis 10 %, vorzugsweise 0,1 bis 1 %<br>99,9 bis 90 %, vorzugsweise 99,9 bis 99 %. |
| Suspensions-Konzentrate: | |
| Wirkstoff der Formel I:<br>Wasser:<br>oberflächenaktives Mittel: | 5 bis 75 %, vorzugsweise 10 bis 50 %<br>94 bis 25 %, vorzugsweise 90 bis 30 %<br>1 bis 40 %, vorzugsweise 2 bis 30 %. |
| Benetzbare Pulver: | |
| Wirkstoff der Formel I:<br>oberflächenaktives Mittel:<br>festes Trägermittel: | 0,5 bis 90 %, vorzugsweise 1 bis 80 %<br>0,5 bis 20 %, vorzugsweise 1 bis 15 %<br>5 bis 95 %, vorzugsweise 15 bis 90 %. |
| Granulate: | |
| Wirkstoff der Formel I:<br>festes Trägermittel: | 0,5 bis 30 %, vorzugsweise 3 bis 15 %<br>99,5 bis 70 %, vorzugsweise 97 bis 85 %. |
| Streugranulate | |
| Wirkstoff der Formel I:<br>Klebstoff:<br>oberflächenaktives Mittel:<br>festes Trägermittel: | 0,01 bis 30 %, vorzugsweise 0,05 bis 15 %<br>0,05 bis 5 %, vorzugsweise 0,1 bis 2%<br>0,5 bis 20%, vorzugsweise 1 bis 15%<br>99,44 bis 45%, vorzugsweise 95 bis 65% |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Augwandmengen betragen in der Regel 0,005 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Herstellungsbeispiele

### H.1. N-(2,6-Dichlorphenyl)-N-(4-trifluormethyl-6-methyl-pyrid in-2-yl)-harnstoff

3,2g (0,01 Mol) 2-(2,6-Dichloranilino)-4-trifluormethyl-6-methyl-pyridin werden in 80 ml Essigsäureethylester gelöst und auf 0 °C gekühlt. Dann werden 1,8 g (0,013Mol) Chlorsulfonylisocyanat zugegeben. Das Reaktionsgemisch wird 2 Stunden bei 0 - 5 °C gerührt. Dann werden 20 ml Eiswasser zugegeben. Nach 30 minütigem Rühren wird die Essigesterphase abgetrennt, mit Sole gewaschen, mit Natriumsulfat getrocknet und eingedampft. Beim Verreiben mit Hexan kristallisiert das Produkt.

Man isoliert 3,0 g (83%) der Titelverbindung der Formel

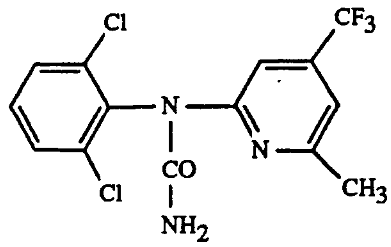

als Kristalle vom Smp. 165 °C (Verb.No. 1.033).

Analog zu vorstehendem Beispiel können die Verbindungen der Tabelle 1 hergestellt werden:

Tabelle 1

Verbindungen der Formel 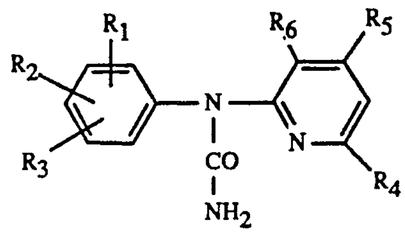

| Verb. No. | $R_1$ | $R_2$ | $R_3$ | $R_4$ | $R_5$ | $R_6$ | Physikal. Daten |
|---|---|---|---|---|---|---|---|
| 1.001 | H | H | H | $CH_3$ | $CF_3$ | H | Fp. 134°C |
| 1.002 | H | H | H | $CH_3$ | $CF_3$ | CN | |
| 1.003 | H | H | H | $CH_3$ | $CH_3$ | CN | |
| 1.004 | 2-Cl | H | H | $CH_3$ | $CH_3$ | H | |
| 1.005 | 2-Cl | H | H | $CH_3$ | $CF_3$ | H | Fp. 122-123°C |
| 1.006 | 2-Cl | H | H | $CH_3$ | Cl | H | |
| 1.007 | 2-Cl | H | H | Cl | $CH_3$ | H | |
| 1.008 | 2-Br | H | H | $CH_3$ | $CF_3$ | H | Fp. 127-128°C |
| 1.009 | 2-Br | H | H | $CF_3$ | $CH_3$ | H | |
| 1.010 | 2-Br | H | H | $C_2H_5$ | $CF_3$ | H | |
| 1.011 | 2-Br | H | H | i-$C_3H_7$ | $CF_3$ | H | |
| 1.012 | 2-Br | H | H | O-$CH_3$ | $CF_3$ | H | |
| 1.013 | 2-Br | H | H | Cl | $CF_3$ | H | |
| 1.014 | 2-Br | H | H | $CH_3$ | $CH_3$ | H | |
| 1.015 | 2-Br | H | H | Cl | $CH_3$ | H | |
| 1.016 | 2-Br | H | H | $CH_3$ | $CF_3$ | CN | |
| 1.017 | 2-$CF_3$ | H | H | Cl | $CH_3$ | H | |

| Verb. No. | $R_1$ | $R_2$ | $R_3$ | $R_4$ | $R_5$ | $R_6$ | Physikal. Daten |
|---|---|---|---|---|---|---|---|
| 1.018 | 2-$CF_3$ | H | H | $CH_3$ | $CF_3$ | H | |
| 1.019 | 2-$CF_3$ | H | H | $CF_3$ | $CH_3$ | H | |
| 1.020 | 2-$CF_3$ | H | H | $C_2H_5$ | $CF_3$ | H | |
| 1.021 | 2-$OCHF_2$ | H | H | $CH_3$ | $CF_3$ | CN | |
| 1.022 | 2-$OCHF_2$ | H | H | $CH_3$ | $CF_3$ | H | |
| 1.023 | 2-$OCHF_2$ | H | H | $C_2H_5$ | $CF_3$ | H | |
| 1.024 | 2-J | H | H | $CH_3$ | $CF_3$ | H | |
| 1.025 | 2-J | H | H | $C_2H_5$ | $CF_3$ | H | |
| 1.026 | 2-F | H | H | $CH_3$ | $CH_3$ | H | |
| 1.027 | 2-F | H | H | $CH_3$ | $CF_3$ | H | |
| 1.028 | 2-Cl | 3-Cl | H | $CH_3$ | $CF_3$ | H | Fp. 125-126°C |
| 1.029 | 2-Cl | 5-Cl | H | $CH_3$ | $CF_3$ | H | Fp. 138°C (Zers.) |
| 1.030 | 2-Cl | 5-Cl | H | $CH_3$ | $CH_3$ | H | |
| 1.031 | 2-Cl | 5-Cl | H | Cl | $CH_3$ | H | |
| 1.032 | 2-Cl | 6-Cl | H | $CH_3$ | $CH_3$ | H | |
| 1.033 | 2-Cl | 6-Cl | H | $CH_3$ | $CF_3$ | H | Fp. 165°C (Zers.) |
| 1.034 | 2-Cl | 6-Cl | H | $CF_3$ | $CH_3$ | H | |
| 1.035 | 2-Cl | 6-Cl | H | $CH_3$ | $CF_3$ | CN | |
| 1.036 | 2-Cl | 6-Cl | H | $CH_3$ | $CF_3$ | $NO_2$ | |
| 1.037 | 2-Cl | 6-Cl | H | $CH_3$ | $CF_3$ | H | |
| 1.038 | 2-Cl | 6-Cl | H | Phenyl | $CF_3$ | H | |
| 1.039 | 2-Cl | 6-Cl | H | 2-Furyl | $CF_3$ | H | |
| 1.040 | 2-Cl | 6-Cl | H | $C_2H_5$ | $CF_3$ | H | |
| 1.041 | 2-Cl | 6-Cl | H | n-$C_3H_7$ | $CF_3$ | H | |
| 1.042 | 2-Cl | 6-Cl | H | i-$C_3H_7$ | $CF_3$ | H | |
| 1.043 | 2-Cl | 6-Cl | H | $CH_3$ | Phenyl | H | |
| 1.044 | 2-Cl | 6-Cl | H | $CF_3$ | H | H | |
| 1.045 | 2-Cl | 6-Cl | H | H | $CF_3$ | H | |
| 1.046 | 2-Cl | 6-Cl | H | $CH_2OCH_3$ | $CF_3$ | H | |
| 1.047 | 2-Cl | 6-Cl | H | $CH_3$ | $CF_2Cl$ | H | Fp. 157-158°C |
| 1.048 | 2-Cl | 6-Cl | H | $C_2H_5$ | $CF_2Cl$ | H | |

| Verb. No. | $R_1$ | $R_2$ | $R_3$ | $R_4$ | $R_5$ | $R_6$ | Physikal. Daten |
|---|---|---|---|---|---|---|---|
| 1.049 | 2-Cl | 6-Cl | H | $CH_3$ | $CHF_2$ | H | |
| 1.050 | 2-Cl | 6-Cl | H | $CH_3$ | $CHCl_2$ | H | |
| 1.051 | 2-Cl | 6-Cl | H | $CH_3$ | Cl | $CH_3$ | |
| 1.052 | 2-Cl | 6-Cl | H | Cl | $CH_3$ | H | |
| 1.053 | 2-Cl | 6-$CH_3$ | H | $CH_3$ | $CF_3$ | CN | |
| 1.054 | 2-Cl | 6-$CH_3$ | H | $CH_3$ | $CF_3$ | H | Fp. 148°C |
| 1.055 | 2-Cl | 6-$CH_3$ | H | $C_2H_5$ | $CF_3$ | H | |
| 1.056 | 2-Cl | 6-$CH_3$ | H | i-$C_3H_7$ | $CF_3$ | H | |
| 1.057 | 2-Cl | 6-$CH_3$ | H | $CH_3$ | $CF_2Cl$ | H | Fp. 166-167°C |
| 1.058 | 2-Cl | 6-$CH_3$ | H | $CH_3$ | $CHF_2$ | H | |
| 1.059 | 2-Cl | 6-$CH_3$ | H | $C_2H_5$ | $CF_2Cl$ | H | |
| 1.060 | 2-Cl | 6-F | H | $CH_3$ | $CF_3$ | H | |
| 1.061 | 2-Cl | 6-F | H | $C_2H_5$ | $CF_3$ | H | |
| 1.062 | 2-Cl | 6-F | H | i-$C_3H_7$ | $CF_3$ | H | |
| 1.063 | 2-Cl | 6-F | H | $CH_3$ | $CH_3$ | H | |
| 1.064 | 2-Cl | 6-Cl | 3-$CH_3$ | $CH_3$ | $CF_3$ | H | |
| 1.065 | 2-Cl | 6-Cl | 3-$CH_3$ | $C_2H_5$ | $CF_3$ | H | |
| 1.066 | 2-Cl | 6-Cl | 3-$CH_3$ | $CF_3$ | $CH_3$ | H | |
| 1.067 | 2-Cl | 6-Cl | 3-Cl | $CH_3$ | $CF_3$ | H | |
| 1.068 | 2-Cl | 6-Cl | 3-Cl | Cl | $CH_3$ | H | |
| 1.069 | 2-Cl | 5-F | H | $CH_3$ | $CF_3$ | H | |
| 1.070 | 2-Cl | 5-F | H | $C_2H_5$ | $CF_3$ | H | |
| 1.071 | 2-F | 5-F | H | $CH_3$ | Cl | H | |
| 1.072 | 2-F | 5-F | H | $CH_3$ | $CF_3$ | H | Fp. 109°C |
| 1.073 | 2-F | 5-F | H | $CF_3$ | $CH_3$ | H | |
| 1.074 | 2-F | 5-F | H | $C_2H_5$ | $CF_3$ | H | |
| 1.075 | 2-F | 5-F | H | i-$C_3H_7$ | $CF_3$ | H | |
| 1.076 | 2-F | 5-F | H | $CH_3$ | $CF_2Cl$ | H | |
| 1.077 | 2-F | 5-F | H | $CH_3$ | $CHF_2$ | H | |
| 1.078 | 2-F | 5-F | H | $CH_3$ | $C_2F_5$ | H | |
| 1.079 | 2-F | 6-F | H | $CH_3$ | $CF_3$ | H | Fp. 153°C |

| Verb. No. | $R_1$ | $R_2$ | $R_3$ | $R_4$ | $R_5$ | $R_6$ | Physikal. Daten |
|---|---|---|---|---|---|---|---|
| 1.080 | 2-F | 6-F | H | $CF_3$ | $CH_3$ | H | |
| 1.081 | 2-F | 6-F | H | $C_2H_5$ | $CF_3$ | H | |
| 1.082 | 2-$NO_2$ | H | H | $CH_3$ | $CF_3$ | H | Fp. 132-3° (Zers.) |
| 1.083 | 2-$NO_2$ | H | H | $C_2H_5$ | $CF_3$ | H | Fp. 114°C |
| 1.084 | 2-$NO_2$ | H | H | i-$C_3H_7$ | $CF_3$ | H | Fp. 119°C |
| 1.085 | 2-$NO_2$ | H | H | n-$C_3H_7$ | $CF_3$ | H | Fp. 116°C |
| 1.086 | 2-$NO_2$ | H | H | i-$C_4H_9$ | $CF_3$ | H | |
| 1.087 | 2-$NO_2$ | H | H | s-$C_4H_9$ | $CF_3$ | H | |
| 1.088 | 2-$NO_2$ | H | H | t-$C_4H_9$ | $CF_3$ | H | |
| 1.089 | 2-$NO_2$ | H | H | n-$C_4H_9$ | $CF_3$ | H | |
| 1.090 | 2-$NO_2$ | H | H | $CH_3$ | $C_2F_5$ | H | |
| 1.091 | 2-$NO_2$ | H | H | $C_2H_5$ | $C_2F_5$ | H | |
| 1.092 | 2-$NO_2$ | H | H | $CH_3$ | $CF_2Cl$ | H | Fp. 141-142 |
| 1.093 | 2-$NO_2$ | H | H | $C_2H_5$ | $CF_2Cl$ | H | |
| 1.094 | 2-$NO_2$ | H | H | $CH_3$ | $CHF_2$ | H | Fp. 149°C |
| 1.095 | 2-$NO_2$ | H | H | $C_2H_5$ | $CHF_2$ | H | |
| 1.096 | 2-$NO_2$ | H | H | $CH_3$ | $CHCl_2$ | H | |
| 1.097 | 2-$NO_2$ | H | H | $C_2H_5$ | $CHCl_2$ | H | |
| 1.098 | 2-$NO_2$ | H | H | $CH_2OCH_3$ | $CF_3$ | H | |
| 1.099 | 2-$NO_2$ | H | H | $CF_3$ | $CH_3$ | H | Fp. 160°C |
| 1.100 | 2-$NO_2$ | H | H | $CF_3$ | H | H | Fp. 74-76°C |
| 1.101 | 2-$NO_2$ | H | H | H | $CF_3$ | H | Fp. 135°C |
| 1.102 | 2-$NO_2$ | H | H | $CH_3$ | $CH_3$ | H | Fp. 154°C |
| 1.103 | 2-$NO_2$ | H | H | $CH_3$ | $CF_3$ | CN | |
| 1.104 | 2-$NO_2$ | H | H | $CH_3$ | $CF_3$ | $NO_2$ | |
| 1.105 | 2-$NO_2$ | H | H | $CH_3$ | $CF_3$ | Cl | |
| 1.106 | 2-$NO_2$ | H | H | $C_2H_5$ | $CF_3$ | CN | |
| 1.107 | 2-$NO_2$ | H | H | $CH_3$ | $CF_3$ | $COOCH_3$ | |

| Verb. No. | $R_1$ | $R_2$ | $R_3$ | $R_4$ | $R_5$ | $R_6$ | Physikal. Daten |
|---|---|---|---|---|---|---|---|
| 1.108 | 2-$NO_2$ | H | H | O-$CH_3$ | $CF_3$ | H | |
| 1.109 | 2-$NO_2$ | H | H | O-$CH_3$ | $CH_3$ | H | |
| 1.110 | 2-$NO_2$ | H | H | CN | $CH_3$ | H | |
| 1.111 | 2-$NO_2$ | H | H | Cl | $CH_3$ | H | Fp. 185°C |
| 1.112 | 2-$NO_2$ | H | H | $CH_3$ | Cl | H | Fp. 146°C |
| 1.113 | 2-$NO_2$ | 6-F | H | $CH_3$ | $CF_3$ | H | Fp. 140°C |
| 1.114 | 2-$NO_2$ | 6-F | H | $C_2H_5$ | $CF_3$ | H | |
| 1.115 | 2-$NO_2$ | 6-F | H | $CF_3$ | $CH_3$ | H | |
| 1.116 | 2-$NO_2$ | 6-F | H | Cl | $CH_3$ | H | |
| 1.117 | 2-$NO_2$ | 6-F | H | $CH_3$ | $CH_3$ | H | |
| 1.118 | 2-$NO_2$ | 6-Cl | H | $CH_3$ | $CF_3$ | H | |
| 1.119 | 2-$NO_2$ | 6-Cl | H | $C_2H_5$ | $CF_3$ | H | |
| 1.120 | 2-$NO_2$ | 6-Cl | H | $CH_3$ | $CF_3$ | CN | |
| 1.121 | 2-$NO_2$ | 6-Cl | H | $CF_3$ | $CH_3$ | H | |
| 1.122 | 2-$NO_2$ | 6-$CH_3$ | H | $CH_3$ | $CF_3$ | H | Fp. 174°C |
| 1.123 | 2-$NO_2$ | 6-$CH_3$ | H | $C_2H_5$ | $CF_3$ | H | Fp. 142-143°C |
| 1.124 | 2-$NO_2$ | 6-$CH_3$ | H | i-$C_3H_7$ | $CF_3$ | H | |
| 1.125 | 2-$NO_2$ | 6-$CH_3$ | H | i-$C_4H_9$ | $CF_3$ | H | |
| 1.126 | 2-$NO_2$ | 6-$CH_3$ | H | $CH_3$ | $CF_2Cl$ | H | Fp. 152-155°C |
| 1.127 | 2-$NO_2$ | 6-$CH_3$ | H | $C_2H_5$ | $CF_2Cl$ | H | |
| 1.128 | 2-$NO_2$ | 6-$CH_3$ | H | i-$C_3H_7$ | $CF_2Cl$ | H | |
| 1.129 | 2-$NO_2$ | 6-$CH_3$ | H | i-$C_4H_9$ | $CF_2Cl$ | H | |
| 1.130 | 2-$NO_2$ | 6-$CH_3$ | H | $CH_3$ | $CHF_2$ | H | Fp. 168°C |
| 1.131 | 2-$NO_2$ | 6-$CH_3$ | H | $C_2H_5$ | $CHF_2$ | H | |
| 1.132 | 2-$NO_2$ | 6-$CH_3$ | H | i-$C_3H_7$ | $CHF_2$ | H | |
| 1.133 | 2-$NO_2$ | 6-$CH_3$ | H | i-$C_4H_9$ | $CHF_2$ | H | |
| 1.134 | 2-$NO_2$ | 6-$CH_3$ | H | $CH_3$ | $CHCl_2$ | H | |
| 1.135 | 2-$NO_2$ | 6-$CH_3$ | H | $C_2H_5$ | $CHCl_2$ | H | |
| 1.136 | 2-$NO_2$ | 6-$CH_3$ | H | i-$C_3H_7$ | $CHCl_2$ | H | |
| 1.137 | 2-$NO_2$ | 6-$CH_3$ | H | i-$C_4H_9$ | $CHCl_2$ | H | |

| Verb. No. | $R_1$ | $R_2$ | $R_3$ | $R_4$ | $R_5$ | $R_6$ | Physikal. Daten |
|---|---|---|---|---|---|---|---|
| 1.138 | 2-$NO_2$ | 6-$CH_3$ | H | $CF_3$ | $CH_3$ | H | |
| 1.139 | 2-$NO_2$ | 6-$CH_3$ | H | $CH_3$ | $CH_3$ | H | |
| 1.140 | 2-$NO_2$ | 6-$CH_3$ | H | Phenyl | $CH_3$ | H | |
| 1.141 | 2-$NO_2$ | 6-$CH_3$ | H | Phenyl | $CF_3$ | H | |
| 1.142 | 2-$NO_2$ | 6-$CH_3$ | H | $CH_3$ | $CF_3$ | CN | |
| 1.143 | 2-$NO_2$ | 6-$CH_3$ | H | $CH_3$ | $CF_3$ | $COOCH_3$ | |
| 1.144 | 2-$NO_2$ | 6-$CH_3$ | H | $CH_3$ | $CF_3$ | Cl | |
| 1.145 | 2-$NO_2$ | 6-$CH_3$ | H | $CH_3$ | $CF_3$ | Br | |
| 1.146 | 2-$NO_2$ | 6-$CH_3$ | H | $CH_3$ | Cl | $CH_3$ | |
| 1.147 | 2-$NO_2$ | 6-$CH_3$ | H | $CH_3$ | $CF_3$ | $NO_2$ | |
| 1.148 | 2-$NO_2$ | 6-$CH_3$ | H | Cl | $CF_3$ | H | |
| 1.149 | 2-$NO_2$ | 6-$CH_3$ | H | Br | $CF_3$ | H | |
| 1.150 | 2-$NO_2$ | 6-$CH_3$ | H | O-$CH_3$ | $CF_3$ | H | |
| 1.151 | 2-$NO_2$ | 5-F | H | $CH_3$ | $CF_3$ | H | Fp. 134°C |
| 1.152 | 2-$NO_2$ | 5-F | H | $CH_3$ | $CF_3$ | CN | |
| 1.153 | 2-$NO_2$ | 5-F | H | $C_2H_5$ | $CF_3$ | H | |
| 1.154 | 2-$NO_2$ | 5-F | H | $CF_3$ | $CF_3$ | H | |
| 1.155 | 2-$NO_2$ | 5-F | H | $CH_3$ | $CH_3$ | H | |
| 1.156 | 2-$NO_2$ | 5-F | H | $C_2H_5$ | $CH_3$ | H | |
| 1.157 | 2-$NO_2$ | 5-F | H | $CF_3$ | $CH_3$ | H | |
| 1.158 | 2-$NO_2$ | 5-F | H | Cl | $CF_3$ | H | |
| 1.159 | 2-$NO_2$ | 5-F | H | Cl | $CH_3$ | H | |
| 1.160 | 2-$OCF_3$ | H | H | $CF_3$ | $CH_3$ | H | |
| 1.161 | 2-$OCF_3$ | H | H | $CH_3$ | $CF_3$ | H | |
| 1.162 | 2-CN | H | H | $CH_3$ | $CF_3$ | H | |
| 1.163 | 2-CN | H | H | $C_2H_5$ | $CF_3$ | H | |
| 1.164 | 2-$NO_2$ | 5-Cl | H | $CH_3$ | $CF_3$ | H | |
| 1.165 | 2-$NO_2$ | 5-Cl | H | $CF_3$ | $CH_3$ | H | |
| 1.166 | 2-$NO_2$ | 5-$CH_3$ | H | $CH_3$ | $CF_3$ | H | |
| 1.167 | 3-Cl | 5-Cl | H | $CH_3$ | $CF_3$ | H | Fp. 142°C |

| Verb. No. | $R_1$ | $R_2$ | $R_3$ | $R_4$ | $R_5$ | $R_6$ | Physikal. Daten |
|---|---|---|---|---|---|---|---|
| 1.168 | 3-Cl | 5-Cl | H | $CH_3$ | $CH_3$ | H | |
| 1.169 | 3-F | H | H | $CH_3$ | $CF_3$ | H | |
| 1.170 | 3-F | H | H | $CF_3$ | $CH_3$ | H | |
| 1.171 | 3-F | H | H | $CH_3$ | $CF_3$ | CN | |
| 1.172 | 3-F | H | H | $CH_3$ | $CH_3$ | H | |
| 1.173 | 3-F | H | H | $CH_3$ | $CH_3$ | CN | |
| 1.174 | 3-Cl | H | H | $CH_3$ | $CF_3$ | H | |
| 1.175 | 3-$CF_3$ | H | H | $CH_3$ | $CF_3$ | H | |
| 1.176 | 3-F | 5-F | H | $CH_3$ | $CF_3$ | H | |
| 1.177 | 2-$NO_2$ | H | H | $CF_3$ | $CF_3$ | H | Fp. 169°C(Zers.) |
| 1.178 | 2-$OCH_3$ | H | H | $CH_3$ | $CF_3$ | H | Fp. 141°C |
| 1.179 | 2-$NO_2$ | H | H | Cl | $CF_3$ | H | Fp. 172°C |
| 1.180 | H | H | H | Cl | $CF_3$ | H | Fp. 115-116°C |
| 1.181 | 2-Cl | 6-Cl | H | Cl | $CF_3$ | H | Fp. 179°C |
| 1.182 | 2-$NO_2$ | 6-$CH_3$ | H | Cl | $CF_3$ | H | Fp. 188°C |
| 1.183 | 2-Cl | H | H | $CH_3$ | $CF_2CL$ | H | Fp. 132-135°C |
| 1.184 | 2-Cl | H | H | $C_2H_5$ | $CF_2Cl$ | H | |
| 1.185 | 2-Cl | H | H | $CH_3$ | $C_2H$ | $H_5$ | |
| 1.186 | 2-Br | H | H | $CH_3$ | $CF_2Cl$ | H | Fp. 137-138°C |
| 1.187 | 2-Br | H | H | $C_2H_5$ | $CF_2Cl$ | H | |
| 1.188 | 2-Br | H | H | $CH_3$ | $CHF_2$ | H | |
| 1.189 | 2-Br | H | H | $CH_3$ | $C_2F_5$ | H | |
| 1.190 | 2-$CF_3$ | H | H | $CH_3$ | $CF_2Cl$ | H | Fp. 131-132°C |
| 1.191 | 2-$CF_3$ | H | H | $CH_3$ | $CHF_2$ | H | |
| 1.192 | 2-Cl | 6-Cl | H | $OCH_3$ | $CF_3$ | H | Fp. 182-183°C |
| 1.193 | 2-Cl | 6-Cl | H | $C_3H_7$(i) | $CF_2Cl$ | H | |
| 1.194 | 2-Cl | 6-Cl | H | $CH_3$ | $C_2F_5$ | H | |
| 1.195 | 2-Cl | 6-Cl | H | $CH_3$ | $CFCl_2$ | H | |
| 1.196 | 2-Cl | 5-Cl | H | $CH_3$ | $CF_2Cl$ | H | Fp. 128-129°C |
| 1.197 | 2-Cl | 5-Cl | H | $C_2H_5$ | $CF_2Cl$ | H | |
| 1.198 | 2-$OCHF_2$ | 6-$CH_3$ | H | $CH_3$ | $CF_3$ | H | Fp. 145°C |
| 1.199 | 2-$OCHF_2$ | 6-$CH_3$ | H | $CH_3$ | $CF_2Cl$ | H | Fp. 124-125°C |

| Verb. No. | $R_1$ | $R_2$ | $R_3$ | $R_4$ | $R_5$ | $R_6$ | Physikal. Daten |
|---|---|---|---|---|---|---|---|
| 1.200 | 2-$OCHF_2$ | 6-$CH_3$ | H | $C_2H_5$ | $CF_2Cl$ | H | |
| 1.201 | 2-$OCHF_2$ | 6-$CH_3$ | H | $C_3H_7$(i) | $CF_2Cl$ | H | |
| 1.202 | 2-$OCHF_2$ | 6-$CH_3$ | H | $CH_3$ | $CHF_2$ | H | |
| 1.203 | 2-$OCHF_2$ | 6-$CH_3$ | H | $CH_3$ | $C_2F_5$ | H | |
| 1.204 | 2-$OCHF_2$ | 6-$CH_3$ | H | $C_2H_5$ | $CF_3$ | H | |
| 1.205 | 2-$OCHF_2$ | 6-$CH_3$ | H | $C_3H_7$(i) | $CF_3$ | H | |
| 1.206 | 2-$NO_2$ | H | H | F | $CH_3$ | H | |
| 1.207 | 2-$NO_2$ | H | H | $CF_3$ | H | $CF_3$ | |
| 1.208 | 2-$NO_2$ | H | H | $CH_3$ | $CH_3$ | CN | Fp. 200-201°C |
| 1.209 | 2-$NO_2$ | H | H | $CH_3$ | $CH_3$ | H | Fp. 172-173°C |
| 1.210 | 2-$NO_2$ | H | H | $CF_3$ | H | CN | |
| 1.211 | 2-$NO_2$ | H | H | $C_3H_7$(i) | $CF_2Cl$ | H | |
| 1.212 | 2-$NO_2$ | H | H | $CH_3$ | $CFCl_2$ | H | |
| 1.213 | 2-$NO_2$ | H | H | $C_2H_5$ | $CFCl_2$ | H | |
| 1.214 | 2-$NO_2$ | 6-F | H | $CH_3$ | $CF_2Cl$ | H | Fp. 145-146°C |
| 1.215 | 2-$NO_2$ | 6-F | H | $C_2H_5$ | $CF_2Cl$ | H | |
| 1.216 | 2-$NO_2$ | 6-F | H | $C_3H_7$(i) | $CF_2Cl$ | H | |
| 1.217 | 2-$NO_2$ | 6-F | H | $CH_3$ | $C_2F_5$ | H | |
| 1.218 | 2-$NO_2$ | 6-F | H | $C_2H_5$ | $C_2H_5$ | H | |
| 1.219 | 2-$NO_2$ | 6-F | H | $CH_3$ | $CHF_2$ | H | |
| 1.220 | 2-$NO_2$ | 6-F | H | $C_2H_5$ | $CHF_2$ | H | |
| 1.221 | 2-$NO_2$ | 6-F | H | $CH_3$ | $CFCl_2$ | H | |
| 1.222 | 2-$NO_2$ | 6-Cl | H | $CH_3$ | $CF_2Cl$ | H | |
| 1.223 | 2-$NO_2$ | 6-Cl | H | $C_2H_5$ | $CF_2Cl$ | H | |
| 1.224 | 2-$NO_2$ | 6-$CH_3$ | H | $CH_3$ | $CFCl_2$ | H | |
| 1.225 | 2-$NO_2$ | 6-$CH_3$ | H | $CH_3$ | $C_2F_5$ | H | |
| 1.226 | 2-$NO_2$ | 6-$CH_3$ | H | $C_2H_5$ | $C_2F_5$ | H | |
| 1.227 | 2-$NO_2$ | 5-Cl | H | $CH_3$ | $CF_2Cl$ | H | |
| 1.228 | 2-$NO_2$ | 5-Cl | H | $C_2H_5$ | $CF_2Cl$ | H | |
| 1.229 | 2-$NO_2$ | 5-Cl | H | $CH_3$ | $CHF_2$ | H | |

| Verb. No. | $R_1$ | $R_2$ | $R_3$ | $R_4$ | $R_5$ | $R_6$ | Physikal. Daten |
|---|---|---|---|---|---|---|---|
| 1.230 | 2-$NO_2$ | H | H | $CH_3$ | CN | H | Fp. 172-173°C |
| 1.231 | 2-$NO_2$ | 6-$C_2H_5$ | H | $CH_3$ | $CF_3$ | H | |
| 1.232 | 2-$NO_2$ | 6-$C_2H_5$ | H | $CH_3$ | $CF_2Cl$ | H | |
| 1.233 | 2-$NO_2$ | 6-$C_2H_5$ | H | $C_2H_5$ | $CF_3$ | H | |

H.2. Herstellung der Zwischenverbindungen

H.2.1. 2-(2,6-Dichloranilino)-4-trifluormethyl-6-methylpyridin

Zu 1,2 g (0,05 Mol) Natriumhydrid in 20 ml Dimethylsulfoxid werden bei 15 - 20 °C 4,0 g (0,025 Mol) 2,6-Dichloranilin in 20 ml Dimethylsulfoxid zugetropft. Nach dem Abklingen der Wasserstoffentwicklung werden 4.5 g (0,025 Mol) 2-Fluor-4-trifluormethyl-6-methylpyridin in 10 ml Dimethylsulfoxid zugetropft, wobei die Temperatur auf 30 °C steigt. Das Reaktionsgemisch wird 2 h bei Raumtemperatur gerührt, mit Eiswasser versetzt und mit Diethylether extrahiert. Die Etherphase wird mit Natriumsulfat getrocknet, eingedampft, der Rückstand an Kieselgel mit Essigsäureethylester I Hexan (1:3) chromatographiert.

Man isoliert 4,4g (54,8%) der Titelverbindung der Formel:

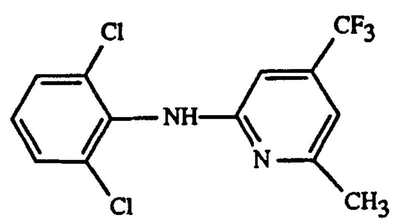

Als Kristalle vom Smp. 108 °C (Verb.No. 2.033)

H.2.2 2-(2-Nitroanilino)-4-methyl-6-chlorpyridin

Zu einer Suspension von 1,4g (0,06 Mol) Natriumhydrid in 20 ml Dimethylsulfoxid werden bei 20 °C 4,3 g (0,03Mol) 2-Amino-4-methyl-6-chlorpyridin in 50 ml DMSO zugetropft. Nach einstündigem Rühren werden 4,2 g (0,03 Mol) 2-Fluor-nitrobenzol in 10 ml DMSO zugetropft, wobei die Temperatur auf 30 °C steigt. Das Reaktionsgemisch wird 2 STunden bei Raumtemperatur gerührt, mit Eiswasser versetzt und mit Essigsäureethylester extrahiert. Der Extrakt wird mit Natriumsulfat getrocknet und eingedampft und der Rückstand danach mit Essigsäureethylester/Hexan (1:3) an Kieselgel chromatographiert.

Man isoliert 5,0 g der Titelverbindung der Formel

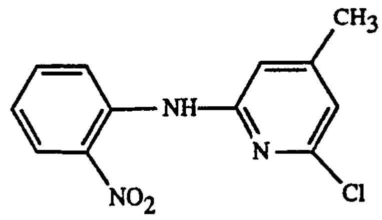

als Kristalle vom Smp. 122 °C (Verb. No. 2.111)

In analoger Weise können die nachstehend in Tabelle 2 genannten Verbindungen der Formel II hergestellt werden:

Tabelle 2

Verbindungen der Formel 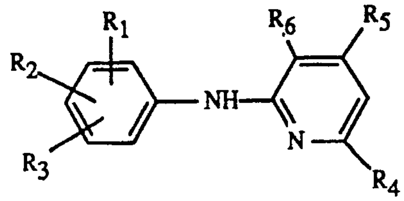 (II)

| Verb. No. | $R_1$ | $R_2$ | $R_3$ | $R_4$ | $R_5$ | $R_6$ | Physikal. Daten |
|---|---|---|---|---|---|---|---|
| 2.001 | H | H | H | $CH_3$ | $CF_3$ | H | $n_D^{25}$ 1.5580 |
| 2.002 | H | H | H | $CH_3$ | $CF_3$ | CN | |
| 2.003 | H | H | H | $CH_3$ | $CH_3$ | CN | |
| 2.004 | 2-Cl | H | H | $CH_3$ | $CH_3$ | H | |
| 2.005 | 2-Cl | H | H | $CH_3$ | $CF_3$ | H | Fp. 53-55°C |
| 2.006 | 2-Cl | H | H | $CH_3$ | Cl | H | |
| 2.007 | 2-Cl | H | H | Cl | $CH_3$ | H | |
| 2.008 | 2-Br | H | H | $CH_3$ | $CF_3$ | H | Fp. 65-67°c |
| 2.009 | 2-Br | H | H | $CF_3$ | $CH_3$ | H | |
| 2.010 | 2-Br | H | H | $C_2H_5$ | $CF_3$ | H | |
| 2.011 | 2-Br | H | H | i-$C_3H_7$ | $CF_3$ | H | |
| 2.012 | 2-Br | H | H | O-$CH_3$ | $CF_3$ | H | |
| 2.013 | 2-Br | H | H | Cl | $CF_3$ | H | |
| 2.014 | 2-Br | H | H | $CH_3$ | $CH_3$ | H | |
| 2.015 | 2-Br | H | H | Cl | $CH_3$ | H | |
| 2.016 | 2-Br | H | H | $CH_3$ | $CF_3$ | CN | |
| 2.017 | 2-$CF_3$ | H | H | Cl | $CH_3$ | H | |
| 2.018 | 2-$CF_3$ | H | H | $CH_3$ | $CF_3$ | H | |
| 2.019 | 2-$CF_3$ | H | H | $CF_3$ | $CH_3$ | H | |

| Verb. No. | $R_1$ | $R_2$ | $R_3$ | $R_4$ | $R_5$ | $R_6$ | Physikal. Daten |
|---|---|---|---|---|---|---|---|
| 2.020 | 2-$CF_3$ | H | H | $C_2H_5$ | $CF_3$ | H | |
| 2.021 | 2-$OCHF_2$ | H | H | $CH_3$ | $CF_3$ | CN | |
| 2.022 | 2-$OCHF_2$ | H | H | $CH_3$ | $CF_3$ | H | |
| 2.023 | 2-$OCHF_2$ | H | H | $C_2H_5$ | $CF_3$ | H | |
| 2.024 | 2-J | H | H | $CH_3$ | $CF_3$ | H | |
| 2.025 | 2-J | H | H | $C_2H_5$ | $CF_3$ | H | |
| 2.026 | 2-F | H | H | $CH_3$ | $CH_3$ | H | |
| 2.027 | 2-F | H | H | $CH_3$ | $CF_3$ | H | |
| 2.028 | 2-Cl | 3-Cl | H | $CH_3$ | $CF_3$ | H | Fp. 91-92°C |
| 2.029 | 2-Cl | 5-Cl | H | $CH_3$ | $CF_3$ | H | Fp. 93°C |
| 2.030 | 2-Cl | 5-Cl | H | $CH_3$ | $CH_3$ | H | |
| 2.031 | 2-Cl | 5-Cl | H | Cl | $CH_3$ | H | |
| 2.032 | 2-Cl | 6-Cl | H | $CH_3$ | $CH_3$ | H | |
| 2.033 | 2-Cl | 6-Cl | H | $CH_3$ | $CF_3$ | H | Fp. 108°C |
| 2.034 | 2-Cl | 6-Cl | H | $CF_3$ | $CH_3$ | H | |
| 2.035 | 2-Cl | 6-Cl | H | $CH_3$ | $CF_3$ | CN | |
| 2.036 | 2-Cl | 6-Cl | H | $CH_3$ | $CF_3$ | $NO_2$ | |
| 2.037 | 2-Cl | 6-Cl | H | $CH_3$ | $CF_3$ | H | |
| 2.038 | 2-Cl | 6-Cl | H | Phenyl | $CF_3$ | H | |
| 2.039 | 2-Cl | 6-Cl | H | 2-Furyl | $CF_3$ | H | |
| 2.040 | 2-Cl | 6-Cl | H | $C_2H_5$ | $CF_3$ | H | |
| 2.041 | 2-Cl | 6-Cl | H | n-$C_3H_7$ | $CF_3$ | H | |
| 2.042 | 2-Cl | 6-Cl | H | i-$C_3H_7$ | $CF_3$ | H | |
| 2.043 | 2-Cl | 6-Cl | H | $CH_3$ | Phenyl | H | |
| 2.044 | 2-Cl | 6-Cl | H | $CF_3$ | H | H | |
| 2.045 | 2-Cl | 6-Cl | H | H | $CF_3$ | H | |
| 2.046 | 2-Cl | 6-Cl | H | $CH_2OCH_3$ | $CF_3$ | H | |
| 2.047 | 2-Cl | 6-Cl | H | $CH_3$ | $CF_2Cl$ | H | Fp. 113-116°C |
| 2.048 | 2-Cl | 6-Cl | H | $C_2H_5$ | $CF_2Cl$ | H | |
| 2.049 | 2-Cl | 6-Cl | H | $CH_3$ | $CHF_2$ | H | |
| 2.050 | 2-Cl | 6-Cl | H | $CH_3$ | $CHCl_2$ | H | |

| Verb. No. | $R_1$ | $R_2$ | $R_3$ | $R_4$ | $R_5$ | $R_6$ | Physikal. Daten |
|---|---|---|---|---|---|---|---|
| 2.051 | 2-Cl | 6-Cl | H | $CH_3$ | Cl | $CH_3$ | |
| 2.052 | 2-Cl | 6-Cl | H | Cl | $CH_3$ | H | |
| 2.053 | 2-Cl | 6-$CH_3$ | H | $CH_3$ | $CF_3$ | CN | |
| 2.054 | 2-Cl | 6-$CH_3$ | H | $CH_3$ | $CF_3$ | H | Oel |
| 2.055 | 2-Cl | 6-$CH_3$ | H | $C_2H_5$ | $CF_3$ | H | |
| 2.056 | 2-Cl | 6-$CH_3$ | H | i-$C_3H_7$ | $CF_3$ | H | |
| 2.057 | 2-Cl | 6-$CH_3$ | H | $CH_3$ | $CF_2Cl$ | H | Fp. 123-124°C |
| 2.058 | 2-Cl | 6-$CH_3$ | H | $CH_3$ | $CHF_2$ | H | |
| 2.059 | 2-Cl | 6-$CH_3$ | H | $C_2H_5$ | $CF_2Cl$ | H | |
| 2.060 | 2-Cl | 6-F | H | $CH_3$ | $CF_3$ | H | |
| 2.061 | 2-Cl | 6-F | H | $C_2H_5$ | $CF_3$ | H | |
| 2.062 | 2-Cl | 6-F | H | i-$C_3H_7$ | $CF_3$ | H | |
| 2.063 | 2-Cl | 6-F | H | $CH_3$ | $CH_3$ | H | |
| 2.064 | 2-Cl | 6-Cl | 3-$CH_3$ | $CH_3$ | $CF_3$ | H | |
| 2.065 | 2-Cl | 6-Cl | 3-$CH_3$ | $C_2H_5$ | $CF_3$ | H | |
| 2.066 | 2-Cl | 6-Cl | 3-$CH_3$ | $CF_3$ | $CH_3$ | H | |
| 2.067 | 2-Cl | 6-Cl | 3-Cl | $CH_3$ | $CF_3$ | H | |
| 2.068 | 2-Cl | 6-Cl | 3-Cl | Cl | $CH_3$ | H | |
| 2.069 | 2-Cl | 5-F | H | $CH_3$ | $CF_3$ | H | |
| 2.070 | 2-Cl | 5-F | H | $C_2H_5$ | $CF_3$ | H | |
| 2.071 | 2-F | 5-F | H | $CH_3$ | Cl | H | |
| 2.072 | 2-F | 5-F | H | $CH_3$ | $CF_3$ | H | Fp. 74°C |
| 2.073 | 2-F | 5-F | H | $CF_3$ | $CH_3$ | H | |
| 2.074 | 2-F | 5-F | H | $C_2H_5$ | $CF_3$ | H | |
| 2.075 | 2-F | 5-F | H | i-$C_3H_7$ | $CF_3$ | H | |
| 2.076 | 2-F | 5-F | H | $CH_3$ | $CF_2Cl$ | H | |
| 2.077 | 2-F | 5-F | H | $CH_3$ | $CHF_2$ | H | |
| 2.078 | 2-F | 5-F | H | $CH_3$ | $C_2F_5$ | H | |
| 2.079 | 2-F | 6-F | H | $CH_3$ | $CF_3$ | H | Fp. 132°C |
| 2.080 | 2-F | 6-F | H | $CF_3$ | $CH_3$ | H | |
| 2.081 | 2-F | 6-F | H | $C_2H_5$ | $CF_3$ | H | |

| Verb. No. | $R_1$ | $R_2$ | $R_3$ | $R_4$ | $R_5$ | $R_6$ | Physikal. Daten |
|---|---|---|---|---|---|---|---|
| 2.082 | 2-$NO_2$ | H | H | $CH_3$ | $CF_3$ | H | Fp. 107-108°C |
| 2.083 | 2-$NO_2$ | H | H | $C_2H_5$ | $CF_3$ | H | Fp. 87°C |
| 2.084 | 2-$NO_2$ | H | H | i-$C_3H_7$ | $CF_3$ | H | Fp. 68-69°C |
| 2.085 | 2-$NO_2$ | H | H | n-$C_3H_7$ | $CF_3$ | H | Fp. 68°C |
| 2.086 | 2-$NO_2$ | H | H | i-$C_4H_9$ | $CF_3$ | H | |
| 2.087 | 2-$NO_2$ | H | H | s-$C_4H_9$ | $CF_3$ | H | |
| 2.088 | 2-$NO_2$ | H | H | t-$C_4H_9$ | $CF_3$ | H | |
| 2.089 | 2-$NO_2$ | H | H | n-$C_4H_9$ | $CF_3$ | H | |
| 2.090 | 2-$NO_2$ | H | H | $CH_3$ | $C_2F_5$ | H | |
| 2.091 | 2-$NO_2$ | H | H | $C_2H_5$ | $C_2F_5$ | H | |
| 2.092 | 2-$NO_2$ | H | H | $CH_3$ | $CF_2Cl$ | H | Fp. 114-115°C |
| 2.093 | 2-$NO_2$ | H | H | $C_2H_5$ | $CF_2Cl$ | H | |
| 2.094 | 2-$NO_2$ | H | H | $CH_3$ | $CHF_2$ | H | Fp.128-129°C |
| 2.095 | 2-$NO_2$ | H | H | $C_2H_5$ | $CHF_2$ | H | |
| 2.096 | 2-$NO_2$ | H | H | $CH_3$ | $CHCl_2$ | H | |
| 2.097 | 2-$NO_2$ | H | H | $C_2H_5$ | $CHCl_2$ | H | |
| 2.098 | 2-$NO_2$ | H | H | $CH_2OCH_3$ | $CF_3$ | H | |
| 2.099 | 2-$NO_2$ | H | H | $CF_3$ | $CH_3$ | H | Fp. 123°C |
| 2.100 | 2-$NO_2$ | H | H | $CF_3$ | H | H | Fp. 109°C |
| 2.101 | 2-$NO_2$ | H | H | H | $CF_3$ | H | Fp. 99°C |
| 2.102 | 2-$NO_2$ | H | H | $CH_3$ | $CH_3$ | H | Fp. 87°C |
| 2.103 | 2-$NO_2$ | H | H | $CH_3$ | $CF_3$ | CN | Fp. 151°C |
| 2.104 | 2-$NO_2$ | H | H | $CH_3$ | $CF_3$ | $NO_2$ | |
| 2.105 | 2-$NO_2$ | H | H | $CH_3$ | $CF_3$ | Cl | |
| 2.106 | 2-$NO_2$ | H | H | $C_2H_5$ | $CF_3$ | CN | |
| 2.107 | 2-$NO_2$ | H | H | $CH_3$ | $CF_3$ | $COOCH_3$ | |
| 2.108 | 2-$NO_2$ | H | H | O-$CH_3$ | $CF_3$ | H | |
| 2.109 | 2-$NO_2$ | H | H | O-$CH_3$ | $CH_3$ | H | |
| 2.110 | 2-$NO_2$ | H | H | CN | $CH_3$ | H | |
| 2.111 | 2-$NO_2$ | H | H | Cl | $CH_3$ | H | Fp. 122°C |
| 2.112 | 2-$NO_2$ | H | H | $CH_3$ | Cl | H | |

| Verb. No. | $R_1$ | $R_2$ | $R_3$ | $R_4$ | $R_5$ | $R_6$ | Physikal. Daten |
|---|---|---|---|---|---|---|---|
| 2.113 | 2-$NO_2$ | 6-F | H | $CH_3$ | $CF_3$ | H | Fp. 95-103°C |
| 2.114 | 2-$NO_2$ | 6-F | H | $C_2H_5$ | $CF_3$ | H | |
| 2.115 | 2-$NO_2$ | 6-F | H | $CF_3$ | $CH_3$ | H | |
| 2.116 | 2-$NO_2$ | 6-F | H | Cl | $CH_3$ | H | |
| 2.117 | 2-$NO_2$ | 6-F | H | $CH_3$ | $CH_3$ | H | |
| 2.118 | 2-$NO_2$ | 6-Cl | H | $CH_3$ | $CF_3$ | H | |
| 2.119 | 2-$NO_2$ | 6-Cl | H | $C_2H_5$ | $CF_3$ | H | |
| 2.120 | 2-$NO_2$ | 6-Cl | H | $CH_3$ | $CF_3$ | CN | |
| 2.121 | 2-$NO_2$ | 6-Cl | H | $CF_3$ | $CH_3$ | H | |
| 2.122 | 2-$NO_2$ | 6-$CH_3$ | H | $CH_3$ | $CF_3$ | H | Fp. 129-130°C |
| 2.123 | 2-$NO_2$ | 6-$CH_3$ | H | $C_2H_5$ | $CF_3$ | H | Fp. 85-86°C |
| 2.124 | 2-$NO_2$ | 6-$CH_3$ | H | i-$C_3H_7$ | $CF_3$ | H | |
| 2.125 | 2-$NO_2$ | 6-$CH_3$ | H | i-$C_4H_9$ | $CF_3$ | H | |
| 2.126 | 2-$NO_2$ | 6-$CH_3$ | H | $CH_3$ | $CF_2Cl$ | H | Fp. 95-98°C |
| 2.127 | 2-$NO_2$ | 6-$CH_3$ | H | $C_2H_5$ | $CF_2Cl$ | H | |
| 2.128 | 2-$NO_2$ | 6-$CH_3$ | H | i-$C_3H_7$ | $CF_2Cl$ | H | |
| 2.129 | 2-$NO_2$ | 6-$CH_3$ | H | i-$C_4H_9$ | $CF_2Cl$ | H | |
| 2.130 | 2-$NO_2$ | 6-$CH_3$ | H | $CH_3$ | $CHF_2$ | H | Fp. 103°C |
| 2.131 | 2-$NO_2$ | 6-$CH_3$ | H | $C_2H_5$ | $CHF_2$ | H | |
| 2.132 | 2-$NO_2$ | 6-$CH_3$ | H | i-$C_3H_7$ | $CHF_2$ | H | |
| 2.133 | 2-$NO_2$ | 6-$CH_3$ | H | i-$C_4H_9$ | $CHF_2$ | H | |
| 2.134 | 2-$NO_2$ | 6-$CH_3$ | H | $CH_3$ | $CHCl_2$ | H | |
| 2.135 | 2-$NO_2$ | 6-$CH_3$ | H | $C_2H_5$ | $CHCl_2$ | H | |
| 2.136 | 2-$NO_2$ | 6-$CH_3$ | H | i-$C_3H_7$ | $CHCl_2$ | H | |
| 2.137 | 2-$NO_2$ | 6-$CH_3$ | H | i-$C_4H_9$ | $CHCl_2$ | H | |
| 2.138 | 2-$NO_2$ | 6-$CH_3$ | H | $CF_3$ | $CH_3$ | H | |
| 2.139 | 2-$NO_2$ | 6-$CH_3$ | H | $CH_3$ | $CH_3$ | H | |
| 2.140 | 2-$NO_2$ | 6-$CH_3$ | H | Phenyl | $CH_3$ | H | |
| 2.141 | 2-$NO_2$ | 6-$CH_3$ | H | Phenyl | $CF_3$ | H | |
| 2.142 | 2-$NO_2$ | 6-$CH_3$ | H | $CH_3$ | $CF_3$ | CN | |
| 2.143 | 2-$NO_2$ | 6-$CH_3$ | H | $CH_3$ | $CF_3$ | $COOCH_3$ | |

| Verb. No. | $R_1$ | $R_2$ | $R_3$ | $R_4$ | $R_5$ | $R_6$ | Physikal. Daten |
|---|---|---|---|---|---|---|---|
| 2.144 | 2-$NO_2$ | 6-$CH_3$ | H | $CH_3$ | $CF_3$ | Cl | |
| 2.145 | 2-$NO_2$ | 6-$CH_3$ | H | $CH_3$ | $CF_3$ | Br | |
| 2.146 | 2-$NO_2$ | 6-$CH_3$ | H | $CH_3$ | Cl | $CH_3$ | |
| 2.147 | 2-$NO_2$ | 6-$CH_3$ | H | $CH_3$ | $CF_3$ | $NO_2$ | |
| 2.148 | 2-$NO_2$ | 6-$CH_3$ | H | Cl | $CF_3$ | H | |
| 2.149 | 2-$NO_2$ | 6-$CH_3$ | H | Br | $CF_3$ | H | |
| 2.150 | 2-$NO_2$ | 6-$CH_3$ | H | O-$CH_3$ | $CF_3$ | H | |
| 2.151 | 2-$NO_2$ | 5-F | H | $CH_3$ | $CF_3$ | H | Fp. 108°C |
| 2.152 | 2-$NO_2$ | 5-F | H | $CH_3$ | $CF_3$ | CN | |
| 2.153 | 2-$NO_2$ | 5-F | H | $C_2H_5$ | $CF_3$ | H | |
| 2.154 | 2-$NO_2$ | 5-F | H | $CF_3$ | $CF_3$ | H | |
| 2.155 | 2-$NO_2$ | 5-F | H | $CH_3$ | $CH_3$ | H | |
| 2.156 | 2-$NO_2$ | 5-F | H | $C_2H_5$ | $CH_3$ | H | |
| 2.157 | 2-$NO_2$ | 5-F | H | $CF_3$ | $CH_3$ | H | |
| 2.158 | 2-$NO_2$ | 5-F | H | Cl | $CF_3$ | H | |
| 2.159 | 2-$NO_2$ | 5-F | H | Cl | $CH_3$ | H | |
| 2.160 | 2-$OCF_3$ | H | H | $CF_3$ | $CH_3$ | H | |
| 2.161 | 2-$OCF_3$ | H | H | $CH_3$ | $CF_3$ | H | |
| 2.162 | 2-CN | H | H | $CH_3$ | $CF_3$ | H | |
| 2.163 | 2-CN | H | H | $C_2H_5$ | $CF_3$ | H | |
| 2.164 | 2-$NO_2$ | 5-Cl | H | $CH_3$ | $CF_3$ | H | |
| 2.165 | 2-$NO_2$ | 5-Cl | H | $CF_3$ | $CH_3$ | H | |
| 2.166 | 2-$NO_2$ | 5-$CH_3$ | H | $CH_3$ | $CF_3$ | H | |
| 2.167 | 3-Cl | 5-Cl | H | $CH_3$ | $CF_3$ | H | Fp. 70-72°C |
| 2.168 | 3-Cl | 5-Cl | H | $CH_3$ | $CH_3$ | H | |
| 2.169 | 3-F | H | H | $CH_3$ | $CF_3$ | H | |
| 2.170 | 3-F | H | H | $CF_3$ | $CH_3$ | H | |
| 2.171 | 3-F | H | H | $CH_3$ | $CF_3$ | CN | |
| 2.172 | 3-F | H | H | $CH_3$ | $CH_3$ | H | |
| 2.173 | 3-F | H | H | $CH_3$ | $CH_3$ | CN | |
| 2.174 | 3-Cl | H | H | $CH_3$ | $CF_3$ | H | |

| Verb. No. | $R_1$ | $R_2$ | $R_3$ | $R_4$ | $R_5$ | $R_6$ | Physikal. Daten |
|---|---|---|---|---|---|---|---|
| 2.175 | 3-$CF_3$ | H | H | $CH_3$ | $CF_3$ | H | |
| 2.176 | 3-F | 5-F | H | $CH_3$ | $CF_3$ | H | |
| 2.177 | 2-$NO_2$ | H | H | $CF_3$ | $CF_3$ | H | Fp. 138-139°C |
| 2.178 | 2-$OCH_3$ | H | H | $CH_3$ | $CF_3$ | H | $n_D^{20}$ 1.5486 |
| 2.179 | 2-$NO_2$ | H | H | Cl | $CF_3$ | H | Fp. 93-96°C |
| 2.180 | H | H | H | Cl | $CF_3$ | H | |
| 2.181 | 2-Cl | 6-Cl | H | Cl | $CF_3$ | H | Fp. 113-114°C |
| 2.182 | 2-$NO_2$ | 6-$CH_3$ | H | Cl | $CF_3$ | H | Fp. 160-161°C |
| 2.183 | 2-Cl | H | H | $CH_3$ | $CF_2CL$ | H | Fp. 62-64°C |
| 2.184 | 2-Cl | H | H | $C_2H_5$ | $CF_2Cl$ | H | |
| 2.185 | 2-Cl | H | H | $CH_3$ | $C_2H$ | $H_5$ | |
| 2.186 | 2-Br | H | H | $CH_3$ | $CF_2Cl$ | H | Fp. 61-62°C |
| 2.187 | 2-Br | H | H | $C_2H_5$ | $CF_2Cl$ | H | |
| 2.188 | 2-Br | H | H | $CH_3$ | $CHF_2$ | H | |
| 2.189 | 2-Br | H | H | $CH_3$ | $C_2F_5$ | H | |
| 2.190 | 2-$CF_3$ | H | H | $CH_3$ | $CF_2Cl$ | H | Fp. 53-55°C |
| 2.191 | 2-$CF_3$ | H | H | $CH_3$ | $CHF_2$ | H | |
| 2.192 | 2-Cl | 6-Cl | H | $OCH_3$ | $CF_3$ | H | $n_D^{25}$ 1.5600 |
| 2.193 | 2-Cl | 6-Cl | H | $C_3H_7$(i) | $CF_2Cl$ | H | |
| 2.194 | 2-Cl | 6-Cl | H | $CH_3$ | $C_2F_5$ | H | |
| 2.195 | 2-Cl | 6-Cl | H | $CH_3$ | $CFCl_2$ | H | |
| 2.196 | 2-Cl | 5-Cl | H | $CH_3$ | $CF_2Cl$ | H | Fp. 101-102°C |
| 2.197 | 2-Cl | 5-Cl | H | $C_2H_5$ | $CF_2Cl$ | H | |
| 2.198 | 2-$OCHF_2$ | 6-$CH_3$ | H | $CH_3$ | $CF_3$ | H | |
| 2.199 | 2-$OCHF_2$ | 6-$CH_3$ | H | $CH_3$ | $CF_2Cl$ | H | Fp. 84-85°C |

| erb. No. | $R_1$ | $R_2$ | $R_3$ | $R_4$ | $R_5$ | $R_6$ | Physikal. Daten |
|---|---|---|---|---|---|---|---|
| 2.200 | 2-$OCHF_2$ | 6-$CH_3$ | H | $C_2H_5$ | $CF_2Cl$ | H | |
| 2.201 | 2-$OCHF_2$ | 6-$CH_3$ | H | $C_3H_7$(i) | $CF_2Cl$ | H | |
| 2.202 | 2-$OCHF_2$ | 6-$CH_3$ | H | $CH_3$ | $CHF_2$ | H | |
| 2.203 | 2-$OCHF_2$ | 6-$CH_3$ | H | $CH_3$ | $C_2F_5$ | H | |
| 2.204 | 2-$OCHF_2$ | 6-$CH_3$ | H | $C_2H_5$ | $CF_3$ | H | |
| 2.205 | 2-$OCHF_2$ | 6-$CH_3$ | H | $C_3H_7$(i) | $CF_3$ | H | |
| 2.206 | 2-$NO_2$ | H | H | F | $CH_3$ | H | Fp. 134°C |
| 2.207 | 2-$NO_2$ | H | H | $CF_3$ | H | $CF_3$ | Fp. 118-119°C |
| 2.208 | 2-$NO_2$ | H | H | $CH_3$ | $CH_3$ | CN | Fp. 199-200°C |
| 2.209 | 2-$NO_2$ | H | H | $CH_3$ | $CH_3$ | H | |
| 2.210 | 2-$NO_2$ | H | H | $CF_3$ | H | CN | Fp. 132-133°C |
| 2.211 | 2-$NO_2$ | H | H | $C_3H_7$(i) | $CF_2Cl$ | H | |
| 2.212 | 2-$NO_2$ | H | H | $CH_3$ | $CFCl_2$ | H | |
| 2.213 | 2-$NO_2$ | H | H | $C_2H_5$ | $CFCl_2$ | H | |
| 2.214 | 2-$NO_2$ | 6-F | H | $CH_3$ | $CF_2Cl$ | H | Fp. 68-69°C |
| 2.215 | 2-$NO_2$ | 6-F | H | $C_2H_5$ | $CF_2Cl$ | H | |
| 2.216 | 2-$NO_2$ | 6-F | H | $C_3H_7$(i) | $CF_2Cl$ | H | |
| 2.217 | 2-$NO_2$ | 6-F | H | $CH_3$ | $C_2F_5$ | H | |
| 2.218 | 2-$NO_2$ | 6-F | H | $C_2H_5$ | $C_2H_5$ | H | |
| 2.219 | 2-$NO_2$ | 6-F | H | $CH_3$ | $CHF_2$ | H | |
| 2.220 | 2-$NO_2$ | 6-F | H | $C_2H_5$ | $CHF_2$ | H | |
| 2.221 | 2-$NO_2$ | 6-F | H | $CH_3$ | $CFCl_2$ | H | |
| 2.222 | 2-$NO_2$ | 6-Cl | H | $CH_3$ | $CF_2Cl$ | H | |
| 2.223 | 2-$NO_2$ | 6-Cl | H | $C_2H_5$ | $CF_2Cl$ | H | |
| 2.224 | 2-$NO_2$ | 6-$CH_3$ | H | $CH_3$ | $CFCl_2$ | H | |
| 2.225 | 2-$NO_2$ | 6-$CH_3$ | H | $CH_3$ | $C_2F_5$ | H | |
| 2.226 | 2-$NO_2$ | 6-$CH_3$ | H | $C_2H_5$ | $C_2F_5$ | H | |
| 2.227 | 2-$NO_2$ | 5-Cl | H | $CH_3$ | $CF_2Cl$ | H | |
| 2.228 | 2-$NO_2$ | 5-Cl | H | $C_2H_5$ | $CF_2Cl$ | H | |
| 2.229 | 2-$NO_2$ | 5-Cl | H | $CH_3$ | $CHF_2$ | H | |

| Verb. No. | $R_1$ | $R_2$ | $R_3$ | $R_4$ | $R_5$ | $R_6$ | Physikal. Daten |
|---|---|---|---|---|---|---|---|
| 2.230 | 2-$NO_2$ | H | H | $CH_3$ | CN | H | |
| 2.231 | 2-$NO_2$ | 6-$C_2H_5$ | H | $CH_3$ | $CF_3$ | H | Fp. 106-108°C |
| 2.232 | 2-$NO_2$ | 6-$C_2H_5$ | H | $CH_3$ | $CF_2Cl$ | H | Fp. 110-113°C |
| 2.233 | 2-$NO_2$ | 6-$C_2H_5$ | H | $C_2H_5$ | $CF_3$ | H | |

## B. Biologische Beispiele

### Beispiel B 1: Pre-emergente Herbizid-Wirkung

Im Gewächshaus wird unmittelbar nach der Einsaat der Versuchspflanzen in Töpfe die Erdoberfläche mit einer wässrigen Dispersion der Wirkstoffe, erhalten aus einem 25 %-igen Emulsionskonzentrat, mit einer Aufwandmenge von 4 kgAS/ha behandelt. Die Töpfe werden im Gewächshaus bei 22-25° C und 50-70 % relativer Luftfeuchtigkeit gehalten und der Versuch nach 3 Wochen ausgewertet.

Die Herbizidwirkung wird dabei in einem neunstufigen (1 = vollständige Schädigung der Versuchspflanze, 9 = keine Herbizidwirkung an der Versuchspflanze) Boniturschema im Vergleich zur unbehandelten Kontrollgruppe ausgewertet.

Boniturnoten von 1 bis 4 (insbesondere von 1 bis 3) weisen auf eine gute bis sehr gute Herbizidwirkung hin.

Die Ergebnisse sind in Tabelle 3 zusammengestellt:

Tabelle 3 :

| Preemergente Herbizidwirkung | | | | |
|---|---|---|---|---|
| Verb. No. | **Testpflanze** | | | |
| | Avena | Sinapis | Setaria | Stellaria |
| 1.083 | 1 | 2 | 1 | 1 |

### Beispiel 82 Postemergente Herbizidwirkung

Eine Anzahl Unkräuter, sowohl monocotyle wie dicotyle, werden nach dem Auflaufen (im 4- bis 6-Blattstadium) mit einer wässrigen Wirkstoffdispersion in einer Dosierung von 4 kgAS/ha auf die Pflanzen gespritzt und diese bei 24 bis 26° C und 40 bis 60 % rel. Luftfeuchtigkeit gehalten. 15 Tage nach der Behandlung wird der Versuch ausgewertet.

Die herbizide Wirkung wird analog zu Beispiel B1 bonitiert.

Die Einzelergebnisse sind in Tabelle 4 zusammengestellt:

Tabelle 4:

| Postemergente Herbizidwirkung | | | | | | |
|---|---|---|---|---|---|---|
| Verb. No. | Testpflanze | | | | | |
| | Avena | Sinapis | Setaria | Stellaria | Lolium | Solanum |
| 1.083 | 4 | 3 | 3 | 4 | 3 | 2 |

B3 Herbizidwirkung geben Unkräuter in Wasserreis

Die Wasserunkräuter werden in Plastikbechern (60 $cm^2$ Oberfläche, 500 ml Volumen) ausgesät. Nach der Saat wird bis zur Erdoberfläche mit Wasser aufgefüllt. 3 Tage nach der Saat wird der Wasserspielgel bis leicht über die Erdoberfläche erhöht (3-5 mm). Die Applikation erfolgt 3 Tage nach der Saat mit einer wässrigen Dispersion der Prüfsubstanz duch eine Spritzung auf die Gefässe. Die Dosis entspricht einer Aufwandmenge von 4 kgAS/ha (Spritzbrühemenge ca 550 l/ha)
Die Pflanzenbecher werden im Gewächshaus unter optimalen Wachstumsbedingungen für die Reisunkräuter aufgestellt, (bei 25 - 30° C und hoher Luftfeuchtigkeit).

Die Auswertung der Versuche findet je nach Wachstumsgeschwindigkeit und Pflanzenart 2 - 3 Wochen nach Applikation statt. Die Bonitur wird analog zu dem in Beispiel 1 genannten Bewertungsschema durchgeführt.

Die Einzelergebnisse sind in Tabelle 5 zusammengestellt:

Tabelle 5

| Herbizidwirkung für Wasserreis | | |
|---|---|---|
| Verb. No. | Testpflanze | |
| | Echinochloa | Monocharia |
| 1.083 | 1 | 1 |

Beispiel B4: Wuchshemmung bei Getreide

Die Pflanzen (z.B. Sommergerste der Sorte Iban) werden in 15 cm-Kunststofftöpfen mit steriler Landerde angesät und in der Klimakammer bei einer Tagestemperatur von 10-15° C und einer Nachttemperatur von 5-10° angezogen. Die Beleuchtungsdauer ist 13,5 Stunden pro Tag.

Ca. 34 Tage nach der Saat und dem Ausdünnen auf 4 Pflanzen/Topf erfolgt die Applikation mit 0,3 bis 3 kg Wirkstoff/ha, in der Regel 25%-ig formuliert und in wässriger Spritzbrühe. Die Wasseraufwandmenge ist ca. 500 l/ha. Nach der Applikation werden die Pflanzen im Gewächshaus bei einer Tagestemperatur von mindestens 10° C aufgestellt. Die Beleuchtungsdauer ist mind. 13,5 Stunden/Tag.

Ca. 28 Tage nach der Behandlung findet die Auswertung statt. Hierbei wird die Höhe des Neuzuwachses dargestellt.

Die geprüften Verbindungen der Formel I bewirken eine Reduzierung des Neuzuwachses im Vergleich zu unbehandelten Kontrolle.

Beispiel 85: Wuchshemmung bei Gräsern mit Klee

Eine Mischung von Gräsern/z.B. Poa, Festuca, Lolium, Bromus, Cynosurus) und Klee (Trifolium pratense/irepens) wird in 15 cm-Kunststofftöpfen mit steriler Landerde angesät und im Gewächshaus bei einer Tagestemperatur von 21° C und einer Nachttemperatur von 17° C angezogen. Die Beleuchungsdauer ist 13,5 Stunden/Tag bei einer Lichtintensität von mind. 7000 Lux. Nach dem Auflauf werden die Pflanzen wöchentlich auf ca. 6 cm Höhe zurückgeschnitten. Ca. 42 Tage nach der Saat und 1 Tag nach dem letzten Schnitt erfolgt die Applikation mit 0,3 bis 3 kg Wirkstoff/ha, in der Regel 25%-ig formulierung und in wässriger Spritzbrühe. Die Wasseraufwandmenge ist ca. 500 l/ha.

Ca. 3 Wochen nach der Behandlung findet die Auswertung statt. Hierbei wird die Höhe des Neuzuwachses gemessen.

Die geprüften Verbindungen der Formel I bewirken eine Reduktion des Neuzuwachses im Vergleich zur unbehandelten Kontrolle.

Beispiel B6: Wuchshemmung bei Getreide

In Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Tabelle 1 besprüht. 21 Tage nach Applikation wird das Wachstum des Getreides beurteilt. Die behandelten Pflanzen weisen im Vergleich zu unbehandelten Kontrollen eine Verringerung des Neuzuwachses, sowie teilweise eine Zunahme der Stengeldurchmesser auf.

Beispiel B7 Wuchshemmung bei Gräsern

In Kunststoffschalen mit Erde-Torf-Sand-Gemisch (6:3:1) werden die Gräser Lolium perenne, Poa pratensis, Fesuca ovina, Dactylis glomerata und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Tabelle 1 besprüht. Die Wirkstoffmenge beträgt umgerechnet bis zu 500 g Aktivsubstanz pro Hektar. 21 Tage nach Applikation wird das Wachstum der Gräser beurteilt.

Die geprüften Verbindungen der Tabelle 1 bewirken eine Reduzierung des Neuzuwachses im Vergleich zur unbehandelten Kontrolle.

Formulierungsbeispiele

Beispiel F1: Formulierungsbeispiele für Wirkstoffe der Formel I′ (% = Gewichtsprozent)

| a) Emulsionskonzentrate | a) | b) | c) |
|---|---|---|---|
| Wirkstoff gemäss Tabelle 1 | 20 % | 40 % | 50 % |
| Ca-Dodecylbenzolsulfonat | 5 % | 8 % | 5,8 % |
| Ricinusöl-polyäthylenglykoläther (36 Mol AeO) | 5 % | - | - |
| Tributylphenol-polyäthylenglykoläther (30 Mol AeO) | - | 12 % | 4,2 % |
| Cyclohexanon | - | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

| b) Lösungen | a) | b) | c) |
|---|---|---|---|
| Wirkstoff gemäss Tabelle 1 | 80 % | 10 % | 5 % |
| Äthylenglykol-monomethyläther | 20 % | - | - |
| Polyäthylenglykol MG 400 | - | 70 % | - |
| N-Methyl-2-pyrrolidon | - | 20 % | 5 % |
| Epoxidiertes Kokosnussöl | - | - | 90 % |

Die Lösungen sind zur Anwendung in Form kleinster Tropfen geeignet.

| c) Granulate | a) | b) |
|---|---|---|
| Wirkstoff gemäss Tabelle 1 | 5 % | 10 % |
| Kaolin | 94 % | - |
| Hochdisperse Kieselsäure | 1 % | |
| Attapulgit | - | 90 % |

Eine Wirkstofflösung wird auf den Träger aufgesprüht und das Lösungsmittel anschliessend im Vakuum abgedampft.

| d) Stäubemittel | a) | b) |
|---|---|---|
| Wirkstoff gemäss Tabelle 1 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

| e) Spritzpulver | a) | b) |
|---|---|---|
| Wirkstoff gemäss Tabelle 1 | 20 % | 60 % |
| Na-Ligninsulfonat | 5 % | 5 % |
| Na-Laurylsulfat | - | 6 % |
| Octylphenolpolyäthylenglykoläther (7-8 Mol Ae) | - | 2 % |
| Hochdisperse Kieselsäure | 5 % | 27 % |
| Kaolin | 70 % | - |

Der Wirkstoff wird mit den Zusatzstoffen gut vermischt und in einer geeigneten Mühle gut vermahlen. Man erhält Spritzpulver, die sich mit Wasser zur Suspension jeder gewünschten Konzentration verdünnen lassen.

| f) Extruder Granulat | |
|---|---|
| Wirkstoff gemäss Tabelle 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses

Gemisch wird extrudiert und anschliessend im Luftstrom getrocknet.

| g) Umhüllungs-Granulat | |
|---|---|
| Wirkstoff gemäss Tabelle 1 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| h) Suspensions-Konzentrat | |
|---|---|
| Wirkstoff gemäss Tabelle 1 | 40 % |
| Äthylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37 %-ige wässrige Formaldehyd-Lösung | 0,2 % |
| Silikonöl in Form einer 75 %-igen wässrigen Emulsion | 0,8 % |
| Wasser | ad 100 % |

Der Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

**Ansprüche**

1. Herbizides oder pflanzenwuchsregulatorisches Mittel, enthaltend als Aktivsubstanz einen Harnstoff der Formel

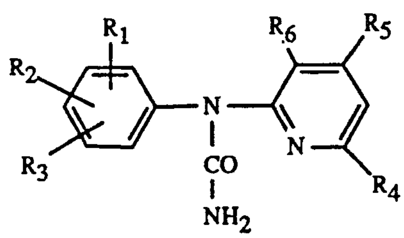

(I),

worin

$R_1$, $R_2$ und $R_3$ unabhängig voneinander Wasserstoff; Nitro; Cyano; Halogen; $C_1$-$C_4$-Alkyl; $C_1$-$C_4$-Alkyl-$S(O)_n$-; $C_1$-$C_4$-Alkoxy; $C_1$-$C_4$-Halogenalkyl; $C_1$-$C_4$-Halogenalkoxy; $C_1$-$C_4$-Halogenalkyl-$S(O)_n$-; $C_1$-$C_4$-Alkoxycarbonyl; $C_1$-$C_4$-Alkylcarbonyl; Aminocarbonyl; Mono-$C_1$-$C_4$-alkylaminocarbonyl; oder Di-$C_1$-$C_4$-alkylaminocarbonyl;

$R_4$ und $R_5$ unabhängig voneinander Wasserstoff; $C_1$-$C_4$-Alkyl; $C_1$-$C_4$-Alkyl-$S(O)_n$-; $C_1$-$C_4$-Alkoxy; $C_1$-$C_4$-Halogenalkyl; $C_1$-$C_4$-Halogenalkoxy; $C_1$-$C_4$-Halogenalkyl-$S(O)_n$-; unsubstituiertes oder bis zu dreifach gleich oder verschieden durch $C_1$-$C_4$-Alkyl, Halogen, $C_1$-$C_4$-Alkoxy, $C_1$-$C_4$-Halogenalkyl, Nitro oder Cyano substituiertes Phenyl; Furanyl; Thiophenyl; $C_3$-$C_6$-Cycloalkyl; $C_1$-$C_4$-Alkoxycarbonyl; $C_1$-$C_4$-Alkoxy-$C_1$-$C_4$-alkyl; $C_1$-$C_4$-Alkoxycarbonyl-$C_1$-$C_4$-alkyl; $C_1$-$C_4$-Alkylcarbonyl-$C_1$-$C_4$-alkyl; $C_3$-$C_4$-Alkenyloxycarbonyl-$C_1$-$C_4$-alkyl; $C_3$-$C_4$-Alkinyloxycarbonyl-$C_1$-$C_4$-alkyl; Halogen; oder Cyano; und

$R_6$ Wasserstoff; $C_1$-$C_4$-Alkyl; Nitro; Cyano; Halogen; $C_1$-$C_4$-Alkoxycarbonyl; $C_1$-$C_4$-Halogenalkyl; und

n 0,1 oder 2

bedeutet unter Einschluss ihrer Salze mit Säuren, Basen und Komplexbildnern, neben üblichen Hilfs-und/oder Trägerstoffen.

2. Mittel gemäss Anspruch 1, enthaltend als Aktivsubstanz eine in der 4-Position des Phenylringes unsubstituierte Verbindung der Formel Ia

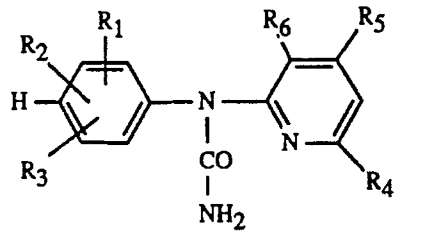

(Ia),

worin die Reste $R_1$ bis $R_6$ wie zuvor definiert sind.

3. Mittel gemäss Anspruch 1 oder 2, enthaltend als Aktivsubstanz eine Verbindungen der Formel Ia

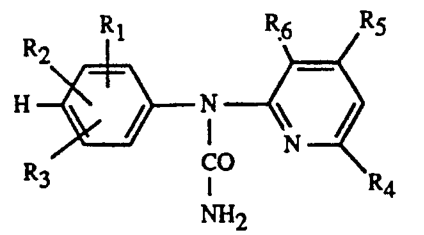

(Ia),

worin
$R_1$, $R_2$ und $R_3$ unabhängig voneinander Wasserstoff; Nitro; Halogen; $C_1$-$C_4$-Alkyl; $C_1$-$C_4$-Halogenalkyl; $C_1$-$C_4$-Alkoxy; oder $C_1$-$C_4$-Halogenalkoxy;
$R_4$ und $R_5$ unabhängig voneinander Wasserstoff; Cyano; Halogen; $C_1$-$C_4$-Alkyl; $C_1$-$C_4$-Alkoxy; $C_1$-$C_4$-Halogenalkyl; $C_1$-$C_4$-Alkoxy-$C_1$-$C_4$-alkyl; Phenyl; oder Furanyl; und
$R_6$ Wasserstoff; Cyano; Nitro; Halogen; oder $C_1$-$C_4$-Alkoxycarbonyl; bedeutet.

4. Mittel gemäss einem oder mehreren der Ansprüche 1 bis 3, enthaltend als Aktivsubstanz eine Verbindung der Formel Ia

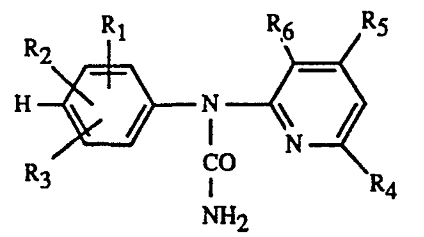

(Ia),

worin
$R_1$ Wasserstoff; Nitro; Halogen; $C_1$-$C_4$-Halogenalkyl; oder $C_1$-$C_4$-Halogenalkoxy;
$R_2$ Wasserstoff; Halogen; oder $C_1$-$C_4$-Alkyl;
$R_3$ Wasserstoff; $C_1$-$C_4$-Alkyl; oder Halogen;
$R_4$ Cyano; Halogen; $C_1$-$C_4$-Alkyl; $C_1$-$C_4$-Alkoxy; $C_1$-$C_4$-Halogenalkyl; $C_1$-$C_4$-Alkoxy-$C_1$-$C_4$-alkyl; Phenyl; oder Furanyl;
$R_5$ Halogen; $C_1$-$C_4$-Alkyl; $C_1$-$C_4$-Halogenalkyl; oder Phenyl; und
$R_6$ Wasserstoff; Cyano; Nitro; Halogen; oder $C_1$-$C_4$-Alkoxycarbonyl
bedeutet.

5. Mittel gemäss einem oder mehreren der Ansprüche 1 bis 4, enthaltend als Aktivsubstanz eine Verbindung der Formel Ia,

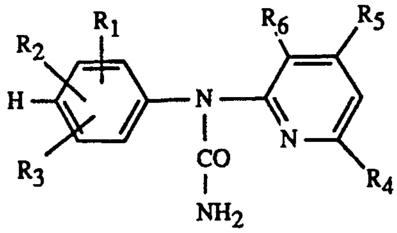

(Ia),

worin

$R_1$ Wasserstoff; Fluor; Chlor; Brom; Jod; Nitro; Trifluormethyl; Methoxy; Trifluormethoxy; oder Difluormethoxy;

$R_2$ Wasserstoff; Fluor; Chlor; oder Methyl;

$R_3$ Wasserstoff; Chlor; oder Methyl;

$R_4$ Chlor; Brom; $C_1$-$C_4$-Alkyl; Cyano; Methoxy; Trifluormethyl; Methoxymethyl; Phenyl; oder Furanyl;

$R_5$ Chlor; Methyl; Trifluormethyl; Chlordifluormethyl; Difluormethyl; Dichlormethyl; oder Pentafluorethyl; und

$R_6$ Wasserstoff; Cyano; Nitro; Chlor; Brom; oder Methoxycarbonyl;

bedeutet.

6. Mittel gemäss einem oder mehreren der Ansprüche 1 bis 5 enthaltend als Aktivsubstanz eine Verbindung der Formel $Ia^1$, $Ia^2$, $Ia^3$, $Ia^4$, $Ia^5$ oder $Ia^6$

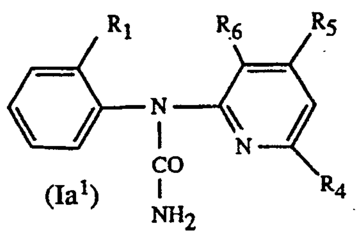

($Ia^1$)

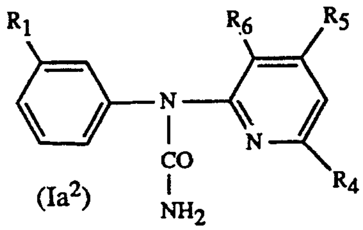

($Ia^2$)

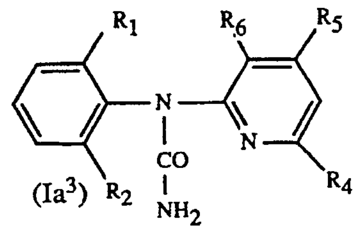

($Ia^3$)

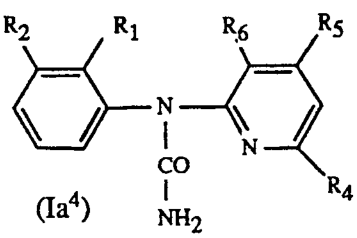

($Ia^4$)

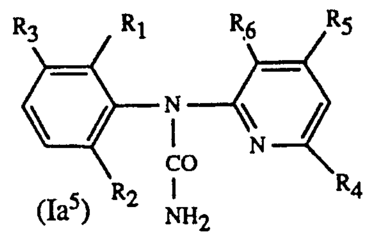

($Ia^5$)

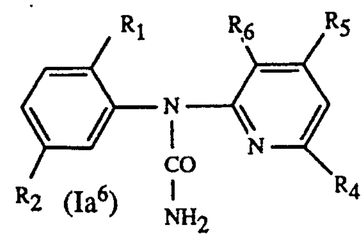

($Ia^6$)

7. Mittel gemäss Anspruch 2, enthaltend als Aktivsubstanz eine Verbindung der Formel Ia, worin die Reste

$R_1$, $R_2$ und $R_3$ wie zuvor definiert sind;

$R_4$ und $R_5$ unabhängig voneinander jeweils Halogen-$C_1$-$C_4$-alkyl; oder $C_1$-$C_4$-Alkyl; und

$R_6$ Wasserstoff;

bedeutet.

8. Mittel gemäss Anspruch 6, enthaltend als Aktivsubstanz eine Verbindung der Formel $Ia^1$ oder $Ia^3$, worin die Reste

$R_1$ und $R_2$ wie zuvor definiert sind;

$R_6$ Wasserstoff; und

$R_4$ und $R_5$ unabhängig voneinander je Halogen-$C_1$-$C_4$-alkyl; oder $C_1$-$C_4$-Alkyl;

bedeutet.

9. Mittel gemäss Anspruch 8 enthaltend als Aktivsubstanz eine Verbindung der Formel $Ia^1$ oder $Ia^3$, worin

$R_1$ Nitro;

$R_2$ wie zuvor definiert ist; und

$R_4$ und $R_5$unabhängig voneinander je Halogen-$C_1$-$C_4$-alkyl, insbesondere Trifluormethyl; oder $C_1$-$C_4$-Alkyl;

bedeutet.

10. Harnstoffe der Formel I

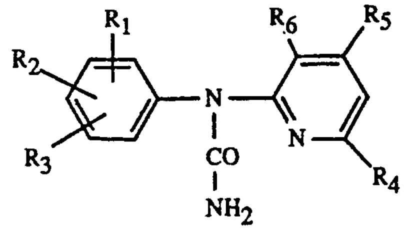

(I),

worin

$R_1$, $R_2$ und $R_3$ unabhängig voneinander Wasserstoff; Nitro; Cyano; Halogen; $C_1$-$C_4$-Alkyl; $C_1$-$C_4$-Alkyl-S(O)$_n$-; $C_1$-$C_4$-Alkoxy; $C_1$-$C_4$-Halogenalkyl; $C_1$-$C_4$-Halogenalkoxy; $C_1$-$C_4$-Halogenalkyl-S(O)$_n$-; $C_1$-$C_4$-Alkoxycarbonyl; $C_1$-$C_4$-Alkylcarbonyl; Aminocarbonyl; Mono-$C_1$-$C_4$-alkylaminnocarbonyl; oder Di-$C_1$-$C_4$-alkylaminocarbonyl;

$R_4$ und $R_5$ unabhängig voneinander Wasserstoff; $C_1$-$C_4$-Alkyl; $C_1$-$C_4$-Alkyl-S(O)$_n$-; $C_1$-$C_4$-Alkoxy; $C_1$-$C_4$-Halogenalkyl; $C_1$-$C_4$-Halogenalkoxy; $C_1$-$C_4$-Halogenalkyl-S(O)$_n$-; unsubstituiertes oder bis zu dreifach gleich oder verschieden durch $C_1$-$C_4$-Alkyl, Halogen, $C_1$-$C_4$-Alkoxy, $C_1$-$C_4$-Halogenalkyl, Nitro oder Cyano substituiertes Phenyl; Furanyl; Thiophenyl; $C_3$-$C_6$-Cycloalkyl; $C_1$-$C_4$-Alkoxycarbonyl; $C_1$-$C_4$-Alkoxy-$C_1$-$C_4$-alkyl; $C_1$-$C_4$-Alkoxycarbonyl-$C_1$-$C_4$-alkyl; $C_1$-$C_4$-Alkylcarbonyl-$C_1$-$C_4$-alkyl; $C_3$-$C_4$-Alkenyloxycarbonyl-$C_1$-$C_4$-alkyl; $C_3$-$C_4$-Alkinyloxycarbonyl-$C_1$-$C_4$-alkyl; Halogen; oder Cyano; und

$R_6$ Wasserstoff; $C_1$-$C_4$-Alkyl; Nitro; Cyano; Halogen; $C_1$-$C_4$-Alkoxycarbonyl; $C_1$-$C_4$-Halogenalkyl; und

n 0,1 oder 2

bedeutet, mit der Massgabe, dass die Reste $R_4$, $R_5$ und $R_6$ nicht alle zugleich für Wasserstoff stehen könnnen,

unter Einschluss ihrer Salze mit Säuren, Basen und Komplexbildnern.

11. In der 4-Position des Phenylringes unsubstituierten Harnstoffe der Formel Ia

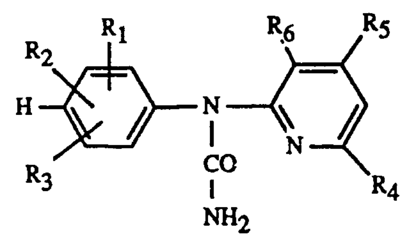

(Ia),

worin die Reste $R_1$ bis $R_6$ wie zuvor definiert sind gemäss Anspruch 10.

12. Harnstoffe der Formel Ia gemäss Anspruch 10 oder 11

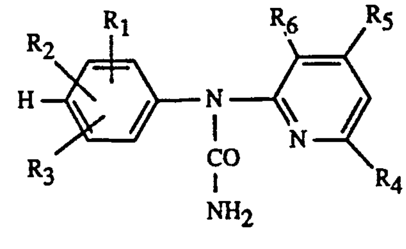

(Ia),

worin

$R_1$, $R_2$ und $R_3$ unabhängig voneinander Wasserstoff; Nitro; Halogen; $C_1$-$C_4$-Alkyl; $C_1$-$C_4$-Halogenalkyl; $C_1$-$C_4$-Alkoxy; oder $C_1$-$C_4$-Halogenalkoxy;

$R_4$ und $R_5$ unabhängig voneinander Wasserstoff; Cyano; Halogen; $C_1$-$C_4$-Alkyl; $C_1$-$C_4$-Alkoxy; $C_1$-$C_4$-Halogenalkyl; $C_1$-$C_4$-Alkoxy-$C_1$-$C_4$-alkyl; Phenyl; oder Furanyl; und

$R_6$ Wasserstoff; Cyano; Nitro; Halogen; oder $C_1$-$C_4$-Alkoxycarbonyl;
bedeutet, enthalten.

13. Harnstoffe der Formel Ia gemäss einem oder mehreren der Ansprüche 10 bis 12

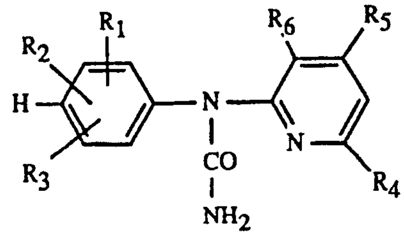

(Ia),

worin
$R_1$ Wasserstoff; Nitro; Halogen; $C_1$-$C_4$-Halogenalkyl; oder $C_1$-$C_4$-Halogenalkoxy;
$R_2$ Wasserstoff; Halogen; oder $C_1$-$C_4$-Alkyl;
$R_3$ Wasserstoff; $C_1$-$C_4$-Alkyl; oder Halogen;
$R_4$ Cyano; Halogen; $C_1$-$C_4$-Alkyl; $C_1$-$C_4$-Alkoxy; $C_1$-$C_4$-Halogenalkyl; $C_1$-$C_4$-Alkoxy-$C_1$-$C_4$-alkyl; Phenyl; oder Furanyl;
$R_5$ Halogen; $C_1$-$C_4$-Alkyl; $C_1$-$C_4$-Halogenalkyl; oder Phenyl; und
$R_6$ Wasserstoff; Cyano; Nitro; Halogen; oder $C_1$-$C_4$-Alkoxycarbonyl
bedeutet.

14. Harnstoffe der Formel Ia, gemäss einem oder mehreren der Ansprüche 10 bis 13

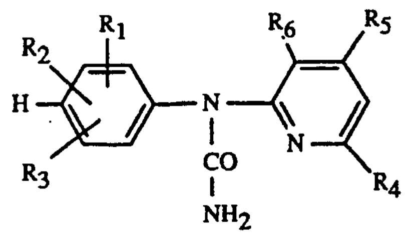

(Ia),

worin
$R_1$ Wasserstoff; Fluor; Chlor; Brom; Jod; Nitro; Trifluormethyl; Methoxy; Trifluormethoxy; oder Difluormethoxy;
$R_2$ Wasserstoff; Fluor; Chlor; oder Methyl;
$R_3$ Wasserstoff; Chlor; oder Methyl;
$R_4$ Chlor; Brom; $C_1$-$C_4$-Alkyl; Cyano; Methoxy; Trifluormethyl; Methoxymethyl; Phenyl; oder Furanyl;
$R_5$ Chlor; Methyl; Trifluormethyl; Chlordifluormethyl; Difluormethyl; Dichlormethyl; oder Pentafluorethyl; und
$R_6$ Wasserstoff; Cyano; Nitro; Chlor; Brom; oder Methoxycarbonyl;
bedeutet.

15. Harnstoffe der Formel $Ia^1$, $Ia^2$, $Ia^3$, $Ia^4$, $Ia^5$ oder $Ia^6$ gemäss einem oder mehreren der Ansprüche 10 bis 14

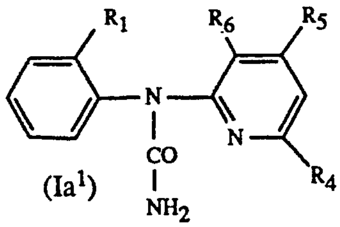
(Ia¹)

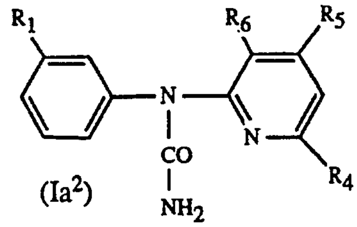
(Ia²)

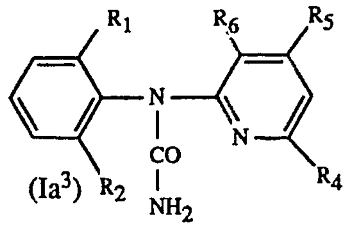
(Ia³)

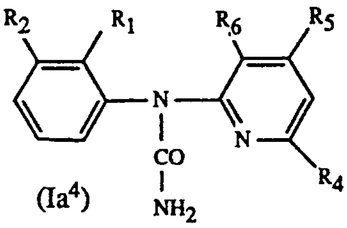
(Ia⁴)

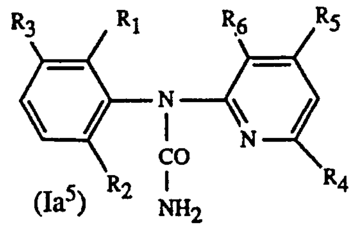
(Ia⁵)

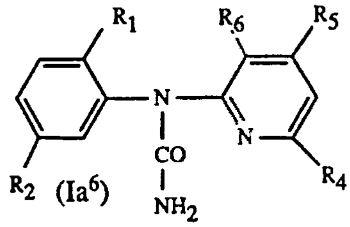
(Ia⁶)

16. Harnstoffe der Formel Ia gemäss einem der Ansprüche 11 bis 15 worin die Reste $R_1$, $R_2$ und $R_3$ wie zuvor definiert sind;
$R_4$ und $R_5$ unabhängig voneinander jeweils Halogen-$C_1$-$C_4$-alkyl; oder $C_1$-$C_4$-Alkyl; und
$R_6$ Wasserstoff;
bedeutet.

17. Harnstoffe der Formel Ia¹ oder Ia³, gemäss Anspruch 15,
worin die Reste
$R_1$ und $R_2$ wie zuvor definiert sind;
$R_6$ Wasserstoff; und
$R_4$ und $R_5$ unabhängig voneinander je Halogen-$C_1$-$C_4$-alkyl; oder $C_1$-$C_4$-Alkyl;
bedeutet.

18. Harnstoffe der Formel Ia¹ oder Ia³, gemäss Anspruch 17, worin
$R_1$ Nitro;
$R_2$ wie zuvor definiert ist; und
$R_4$ und $R_5$ unabhängig voneinander je Halogen-$C_1$-$C_4$-alkyl; oder $C_1$-$C_4$-Alkyl;
bedeutet.

19. Verfahren zur Herstellung von Verbindungen der Formel I gemäss einem oder mehreren der Ansprüche 10 bis 18, dadurch gekennzeichnet, dass man
ein Anilin der Formel II, worin die Reste $R_1$ bis $R_6$ wie unter Formel I definiert sind, mit Phosgen zu einem Carbaminchlorid der Formel III umsetzt und dieses in einer zweiten Stufe mit $NH_3$ zu einem Harnstoff der Formel I umsetzt

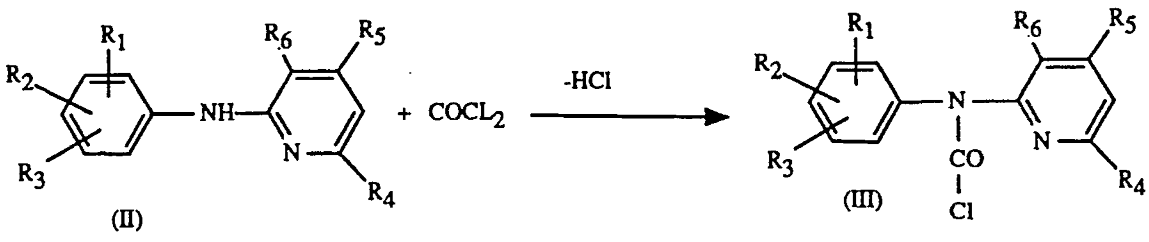

(II) (III)

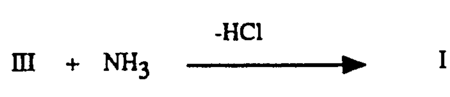


20. Verfahren zur Herstellung von Verbindungen der Formel I,

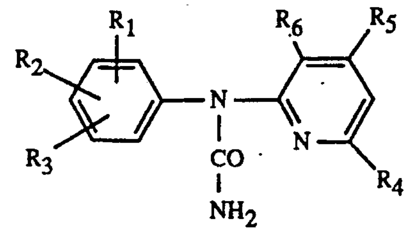

(I),

worin die Reste $R_1$ bis $R_6$ wie in einem der Ansprüche 1 bis 9 definiert sind, dadurch gekennzeichnet, dass man
ein Anilin der Formel II, worin die Reste $R_1$ bis $R_6$ wie unter Formel I definiert sind, mit Halogensulfonylisocyanat V zu einem Halogensulfonylharnstoff der Formel IV umsetzt und diesen dann in einer zweiten Stufe oder direkt zu einer Verbindung der Formel I hydrolisiert, wobei Y für eine unter den Reaktionsbedingungen abspaltbare Gruppe, Wie Halogen, vorzugsweise Chlor, steht:

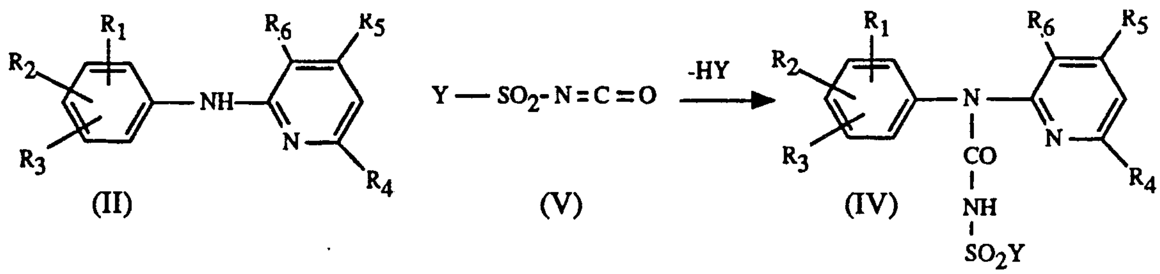

(II) (V) (IV)

$$IV + H_2O \xrightarrow[- SO_4H_2]{- HY} I$$

21. Verfahren zur Herstellung von Harnstoffen der Formel I′

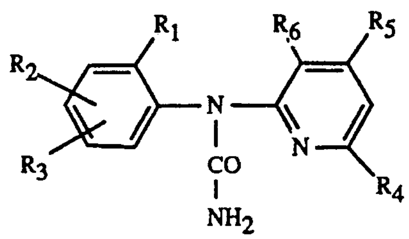

(I′), ($R^1 = NO_2$)

worin $R^1$ in der ortho-Stellung des Phenylringes gebunden ist und Nitro bedeutet und die Reste $R_2$ bis $R_6$ wie unter Formel I in einem der Ansprüche 1 bis 9 definiert sind, dadurch gekennzeichnet, dass man
einen Sulfonylharnstoff der Formel VI unter Einwirkung einer wässrigen Base zu einem Harnstoff der Formel I′ umlagert.

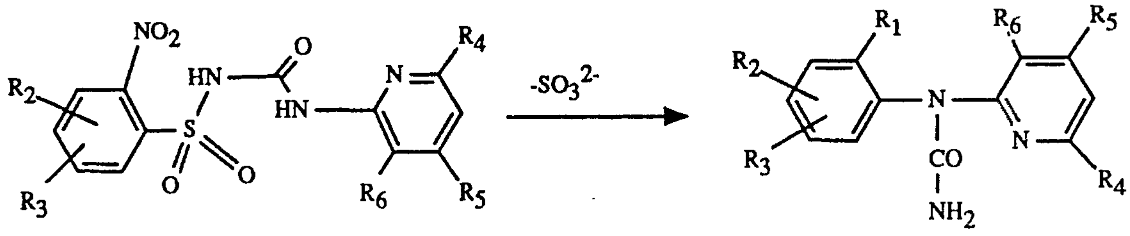

(VI) (I') ($R^1 = NO_2$)

22. Aniline der Formel II

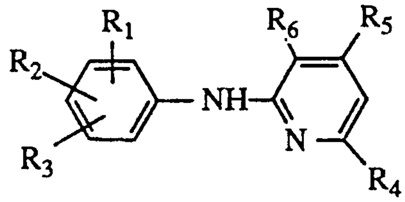

(II)

worin die Reste
$R_1$ bis $R_6$ wie unter Formel I in einem der Ansprüche 10 bis 18 definiert sind.

23. Aniline der Formel $IIa^1$ oder $IIa^3$

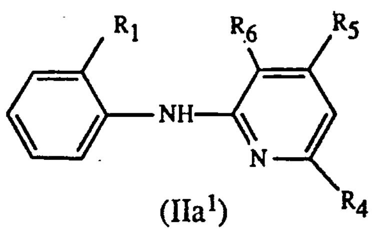

($IIa^1$)

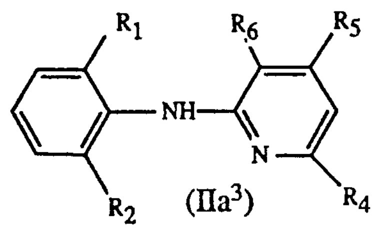

($IIa^3$)

gemäss Anspruch 22.

24. Aniline der Formel $IIa^1$ oder $IIa^3$, gemäss Anspruch 23 worin die Reste
$R_1$ und $R_2$ wie zuvor definiert sind;
$R_6$ Wasserstoff; und
$R_4$ und $R_5$ unabhängig voneinander je Halogen-$C_1$-$C_4$-alkyl, oder $C_1$-$C_4$-Alkyl;
bedeutet.

25. Aniline gemäss Anspruch 24, worin
$R_1$ Nitro;
$R_2$ wie zuvor definiert ist; und
$R_4$ und $R_5$unabhängig voneinander je Halogen-$C_1$-$C_4$-alkyl, ;oder $C_1$-$C_4$-Alkyl;
bedeutet.

26. Verfahren zur Herstellung von Anilinen der Formel II gemäss einem der Ansprüche 22 bis 25, dadurch gekennzeichnet, dass man

a) ein Anilin der Formel VII, worin die Reste $R_1$ bis $R_3$ wie zuvor definiert sind, mit einem Pyridin der Formel III, worin die Reste $R_4$ bis $R_6$ wie unter Formel II definiert sind und X Halogen, $C_1$-$C_4$-Alkyl-$SO_2$-, oder Phenyl-$SO_2$- bedeutet, unter Baseneinwirkung umsetzt

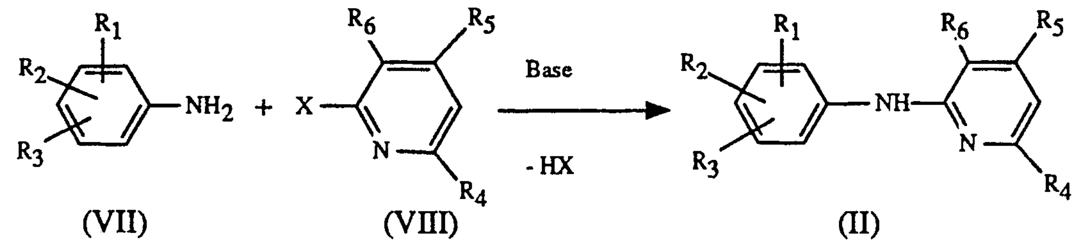

(VII) (VIII) (II)

oder

b) ein Halogenbezol der Formel IX, worin die Reste $R_1$, $R_2$ und $R_3$ wie unter Formel II definiert sind und Y Halogen bedeutet, unter Baseneinwirkung mit einem 2-Aminopyridin der Formel X umsetzt

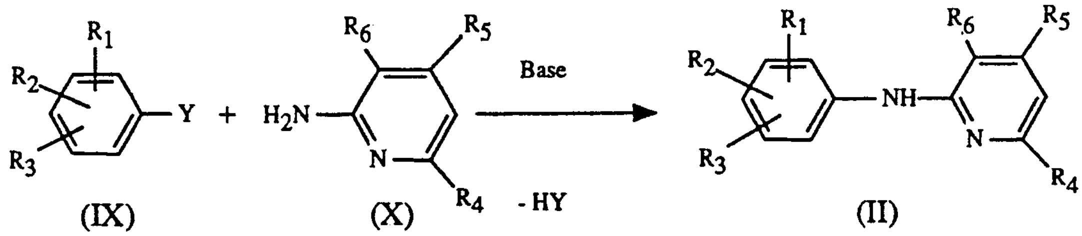

(IX) (X) (II)

27. Carbaminchloride der Formel III

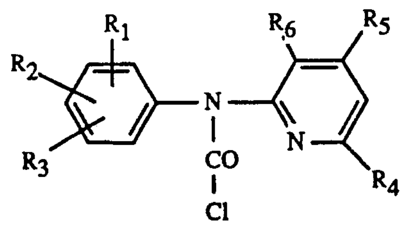
(III),

worin die Reste $R_1$ bis $R_6$ wie in einem der Ansprüche 10 bis 18 unter Formel I definiert sind.

28. Verfahren zur Herstellung von Verbindungen der Fomel III, gemäss Anspruch 27, dadurch gekennzeichnet, dass man ein Anilin der Formel II mit Phosgen umsetzt

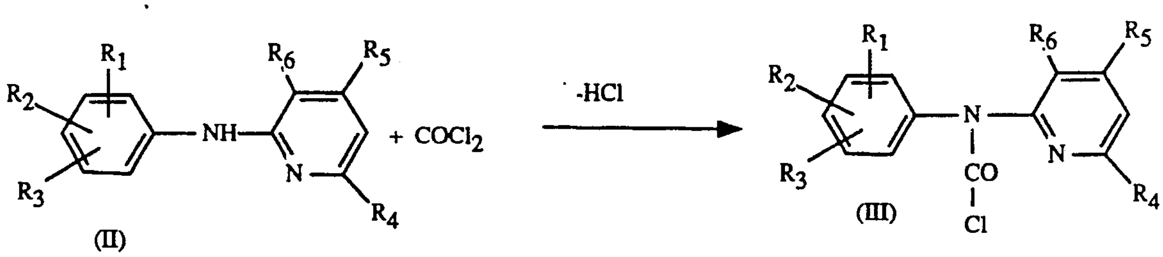

(II) (III)

29. Halogensulfonylharnstoffe der Formel IV

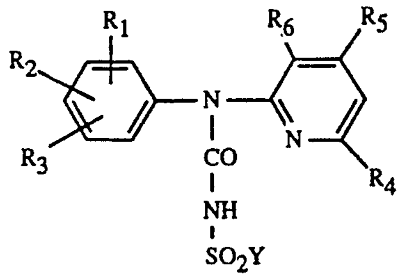

(IV),

worin die Reste $R_1$ bis $R_6$ wie in einem der Ansprüche 1 bis 9 unter Formel I definiert sind.

30. Verfahren zur Herstellung von Verbindungen der Formel IV, gemäss Anspruch 29, dadurch gekennzeichnet, dass man ein Anilin der Formel II, worin die Reste $R_1$ bis $R_6$ wie unter Formel IV definiert sind, mit einem Halogensulfonylisocyanat der Formel V, worin Y für Halogen steht um setzt

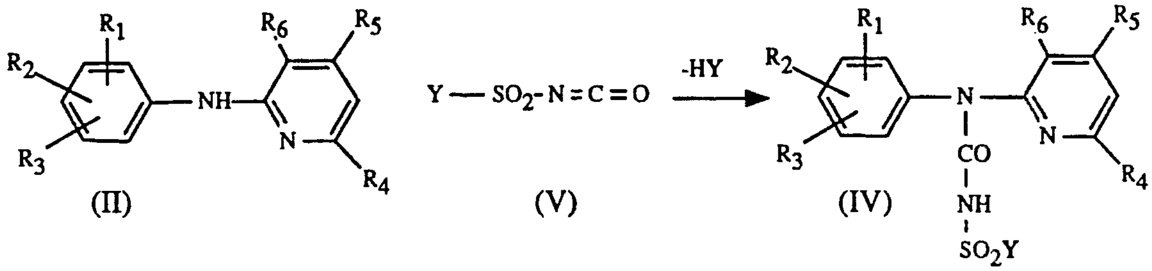


(II) (V) (IV)

31. Verfahren zur Bekämpfung unerwünschten Pflanzenwuchses, dadurch gekennzeichnet, dass man eine herbizid wirksame Menge einer Verbindung der Formel I gemäss einem der Ansprüche 10 bis 18 oder eines Mittels, gemäss einem der Ansprüche 1 bis 9 auf die zu bekämpfende Pflanze oder deren Lebensraum einwirken lässt.

32. Verfahren gemäss Anspruch 31 zur pre- oder postemergenten Bekämpfung unerwünschten Pflanzenwuchses in Nutzpflanzenkulturen.

33. Verfahren zur Beeinflussung des Pflanzenwuchses, dadurch gekennzeichnet, dass man eine pflanzenwuchsregulatorisch wirksame Menge einer Verbindung der Formel I gemäss einem der Ansprüche 10 bis 18 oder eines Mittels gemäss einem der Ansprüche 1 bis 9 auf die Pflanze oder deren Lebensraum einwirken lässt.

34. Saatgut, welches mit einer herbizid oder pflanzenwuchsregulatorisch wirksamen Menge einer Verbindung der Formel I gemäss einem der Ansprüche 10 bis 18 oder einem Mittel gemäss einem der Ansprüche 1 bis 9 behandelt worden ist.

Patentansprüche für folgenden Vertragsstaat: ES

1. Herbizides oder pflanzenwuchsregulatorisches Mittel, enthaltend als Aktivsubstanz einen Harnstoff der Formel I

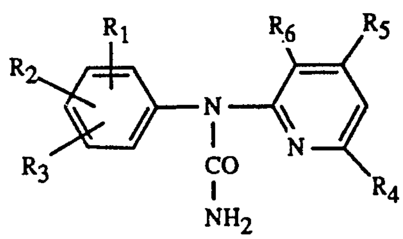

(I),

worin

$R_1$, $R_2$ und $R_3$ unabhängig voneinander Wasserstoff; Nitro; Cyano; Halogen; $C_1$-$C_4$-Alkyl; $C_1$-$C_4$-Alkyl-$S(O)_n$-; $C_1$-$C_4$-Alkoxy; $C_1$-$C_4$-Halogenalkyl; $C_1$-$C_4$-Halogenalkoxy; $C_1$-$C_4$-Halogenalkyl-$S(O)_n$-; $C_1$-$C_4$-Alkoxycarbonyl; $C_1$-$C_4$-Alkylcarbonyl; Aminocarbonyl; Mono-$C_1$-$C_4$-alkylaminocarbonyl; oder Di-$C_1$-$C_4$-alkylaminocarbo-

nyl;
$R_4$ und $R_5$ unabhängig voneinander Wasserstoff; $C_1$-$C_4$-Alkyl; $C_1$-$C_4$-Alkyl-S(O)$_n$-; $C_1$-$C_4$-Alkoxy; $C_1$-$C_4$-Halogenalkyl; $C_1$-$C_4$-Halogenalkoxy; $C_1$-$C_4$-Halogenalkyl-S(O)$_n$-; unsubstituiertes oder bis zu dreifach gleich oder verschieden durch $C_1$-$C_4$-Alkyl, Halogen, $C_1$-$C_4$-Alkoxy, $C_1$-$C_4$-Halogenalkyl, Nitro oder Cyano substituiertes Phenyl; Furanyl; Thiophenyl; $C_3$-$C_6$-Cycloalkyl; $C_1$-$C_4$-Alkoxycarbonyl; $C_1$-$C_4$-Alkoxy-$C_1$-$C_4$-alkyl; $C_1$-$C_4$-Alkoxycarbonyl-$C_1$-$C_4$-alkyl; $C_1$-$C_4$-Alkylcarbonyl-$C_1$-$C_4$-alkyl; $C_3$-$C_4$-Alkenyloxycarbonyl-$C_1$-$C_4$-alkyl; $C_3$-$C_4$-Alkinyloxycarbonyl-$C_1$-$C_4$-alkyl; Halogen; oder Cyano; und
$R_6$ Wasserstoff; $C_1$-$C_4$-Alkyl; Nitro; Cyano; Halogen; $C_1$-$C_4$-Alkoxycarbonyl; $C_1$-$C_4$-Halogenalkyl; und
n 0,1 oder 2
bedeutet unter Einschluss ihrer Salze mit Säuren, Basen und Komplexbildnern, neben üblichen Hilfs- und/oder Trägerstoffen.

2. Mittel gemäss Anspruch 1, enthaltend als Aktivsubstanz eine in der 4-Position des Phenylringes unsubstituierte Verbindung der Formel Ia

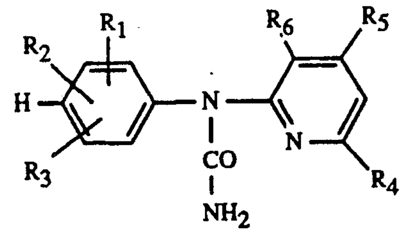
(Ia),

worin die Reste $R_1$ bis $R_6$ wie zuvor definiert sind.

3. Mittel gemäss Anspruch 1 oder 2, enthaltend als Aktivsubstanz eine Verbindungen der Formel Ia

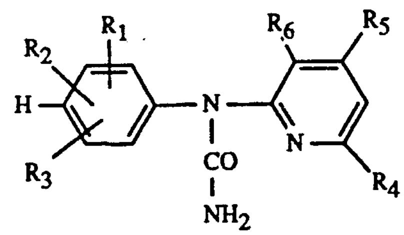
(Ia),

worin
$R_1$, $R_2$ und $R_3$ unabhängig voneinander Wasserstoff; Nitro; Halogen; $C_1$-$C_4$-Alkyl; $C_1$-$C_4$-Halogenalkyl; $C_1$-$C_4$-Alkoxy; oder $C_1$-$C_4$-Halogenalkoxy;
$R_4$ und $R_5$ unabhängig voneinander Wasserstoff; Cyano; Halogen; $C_1$-$C_4$-Alkyl; $C_1$-$C_4$-Alkoxy; $C_1$-$C_4$-Halogenalkyl; $C_1$-$C_4$-Alkoxy-$C_1$-$C_4$-alkyl; Phenyl; oder Furanyl; und
$R_6$ Wasserstoff; Cyano; Nitro; Halogen; oder $C_1$-$C_4$-Alkoxycarbonyl;
bedeutet.

4. Mittel gemäss einem oder mehreren der Ansprüche 1 bis 3, enthaltend als Aktivsubstanz eine Verbindung der Formel Ia

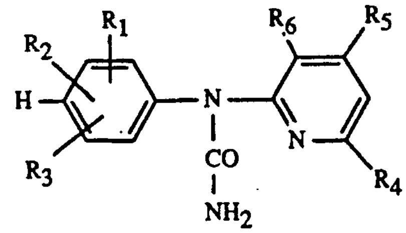
(Ia),

worin
$R_1$ Wasserstoff; Nitro; Halogen; $C_1$-$C_4$-Halogenalkyl; oder $C_1$-$C_4$-Halogenalkoxy;
$R_2$ Wasserstoff; Halogen; oder $C_1$-$C_4$-Alkyl;

$R_3$ Wasserstoff; $C_1$-$C_4$-Alkyl; oder Halogen;
$R_4$ Cyano; Halogen; $C_1$-$C_4$-Alkyl; $C_1$-$C_4$-Alkoxy; $C_1$-$C_4$-Halogenalkyl; $C_1$-$C_4$-Alkoxy-$C_1$-$C_4$-alkyl; Phenyl; oder Furanyl;
$R_5$ Halogen; $C_1$-$C_4$-Alkyl; $C_1$-$C_4$-Halogenalkyl; oder Phenyl; und
$R_6$ Wasserstoff; Cyano; Nitro; Halogen; oder $C_1$-$C_4$-Alkoxycarbonyl
bedeutet.

5. Mittel gemäss einem oder mehreren der Ansprüche 1 bis 4, enthaltend als Aktivsubstanz eine Verbindung der Formel Ia,

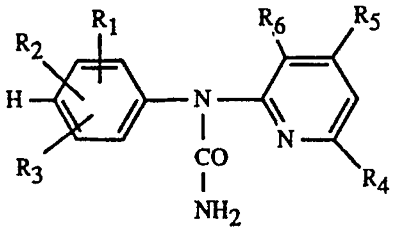
(Ia),

worin
$R_1$ Wasserstoff; Fluor; Chlor; Brom; Jod; Nitro; Trifluormethyl; Methoxy; Trifluormethoxy; oder Difluormethoxy;
$R_2$ Wasserstoff; Fluor; Chlor; oder Methyl;
$R_3$ Wasserstoff; Chlor; oder Methyl;
$R_4$ Chlor; Brom; $C_1$-$C_4$-Alkyl; Cyano; Methoxy; Trifluormethyl; Methoxymethyl; Phenyl; oder Furanyl;
$R_5$ Chlor; Methyl; Trifluormethyl; Chlordifluormethyl; Difluormethyl; Dichlormethyl; oder Pentafluorethyl; und
$R_6$ Wasserstoff; Cyano; Nitro; Chlor; Brom; oder Methoxycarbonyl;
bedeutet.

6. Mittel gemäss einem oder mehreren der Ansprüche 1 bis 5 enthaltend als Aktivsubstanz eine Verbindung der Formel $Ia^1$, $Ia^2$, $Ia^3$, $Ia^4$, $Ia^5$ oder $Ia^6$

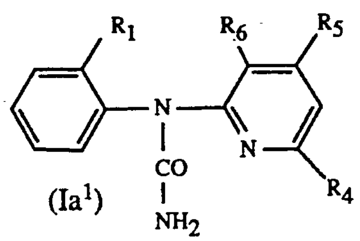
($Ia^1$)

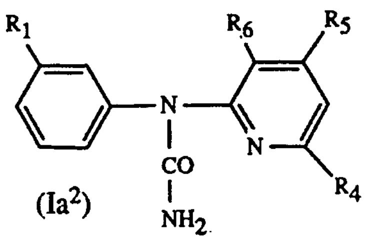
($Ia^2$)

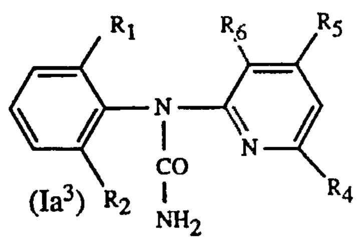
($Ia^3$)

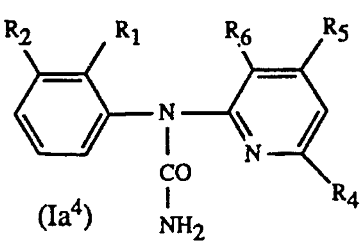
($Ia^4$)

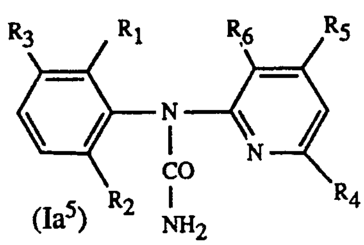
($Ia^5$)

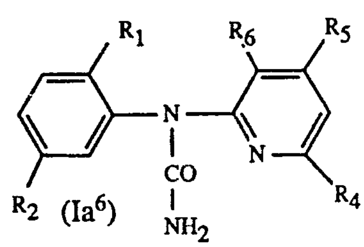
($Ia^6$)

7. Mittel gemäss Anspruch 2, enthaltend als Aktivsubstanz eine Verbindung der Formel Ia, worin die Reste
$R_1$, $R_2$ und $R_3$ wie zuvor definiert sind;
$R_4$ und $R_5$ unabhängig voneinander jeweils Halogen-$C_1$-$C_4$-alkyl; oder $C_1$-$C_4$-Alkyl; und
$R_6$ Wasserstoff;
bedeutet.

8. Mittel gemäss Anspruch 6, enthaltend als Aktivsubstanz eine Verbindung der Formel $Ia^1$ oder $Ia^3$, worin die Reste

$R_1$ und $R_2$ wie zuvor definiert sind;
$R_6$ Wasserstoff; und
$R_4$ und $R_5$ unabhängig voneinander je Halogen-$C_1$-$C_4$-alkyl; oder $C_1$-$C_4$-Alkyl;
bedeutet.

9. Mittel gemäss Anspruch 8 enthaltend als Aktivsubstanz eine Verbindung der Formel Ia[1] oder Ia[3], worin
$R_1$ Nitro;
$R_2$ wie zuvor definiert ist; und
$R_4$ und $R_5$ unabhängig voneinander je Halogen-$C_1$-$C_4$-alkyl, insbesondere Trifluormethyl; oder $C_1$-$C_4$-Alkyl;
bedeutet.

10. Verfahren zur Herstellung von Verbindungen der Formel I gemäss Anspruch 1,
dadurch gekennzeichnet, dass man
ein Anilin der Formel II, worin die Reste $R_1$ bis $R_6$ wie unter Formel I im Anspruch 1 definiert sind, mit Phosgen zu einem Carbaminchlorid der Formel III umsetzt und dieses in einer zweiten Stufe mit $NH_3$ zu einem Harnstoff der Formel I umsetzt

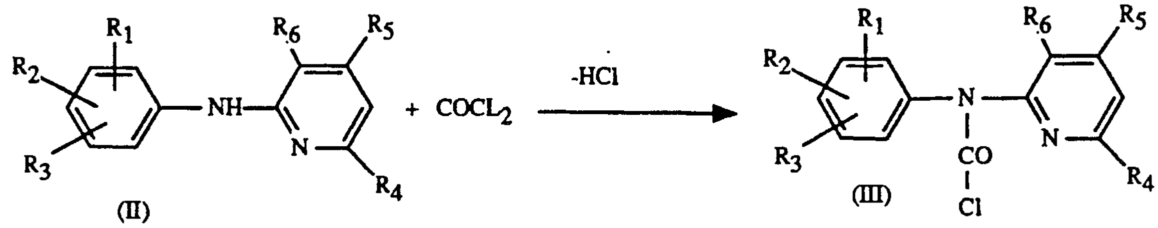


(II) (III)

$$III + NH_3 \xrightarrow{-HCl} I$$

11. Verfahren zur Herstellung von Verbindungen der Formel I,

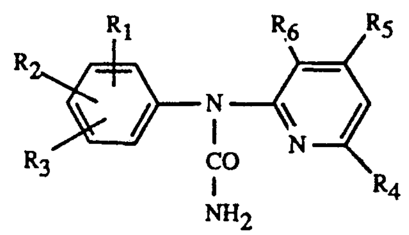

(I),

worin die Reste $R_1$ bis $R_6$ wie unter Formel I in Anspruch 1 definiert sind, dadurch gekennzeichnet, dass man
ein Anilin der Formel II, worin die Reste $R_1$ bis $R_6$ wie unter Formel I in Anspruch 1 definiert sind, mit Halogensulfonylisocyanat V zu einem Halogensulfonylharnstoff der Formel IV umsetzt und diesen dann in einer zweiten Stufe oder direkt zu einer Verbindung der Formel I hydrolisiert, wobei Y für eine unter den Reaktionsbedingungen abspaltbare Gruppe, wie Halogen, vorzugsweise Chlor, steht:

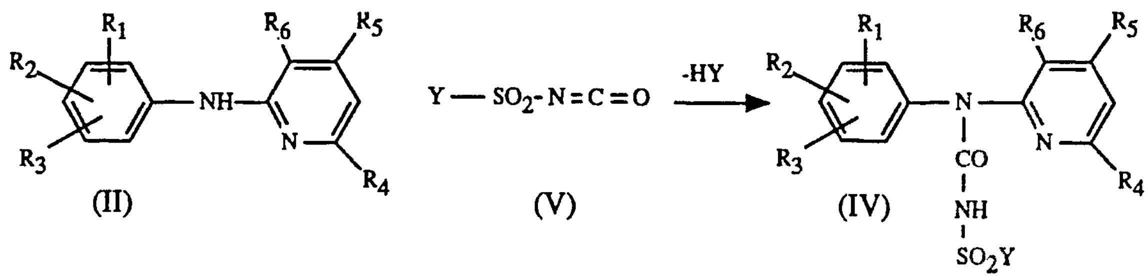


(II) (V) (IV)

$$IV + H_2O \xrightarrow[-SO_4H_2]{-HY} I$$

12. Verfahren zur Herstellung von Harnstoffen der Formel I′

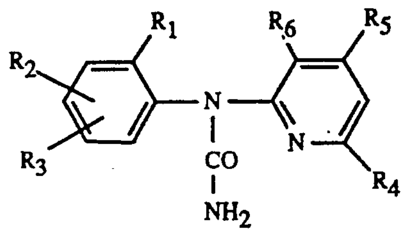

(I'), ($R^1 = NO_2$)

worin $R_1$ in der ortho-Stellung des Phenylringes gebunden ist und Nitro bedeutet und die Reste $R_2$ bis $R_6$ wie unter Formel I in Anspruch 1 definiert sind, dadurch gekennzeichnet, dass man einen Sulfonylharnstoff der Formel VI unter Einwirkung einer wässrigen Base zu einem Harnstoff der Formel I′ umlagert.

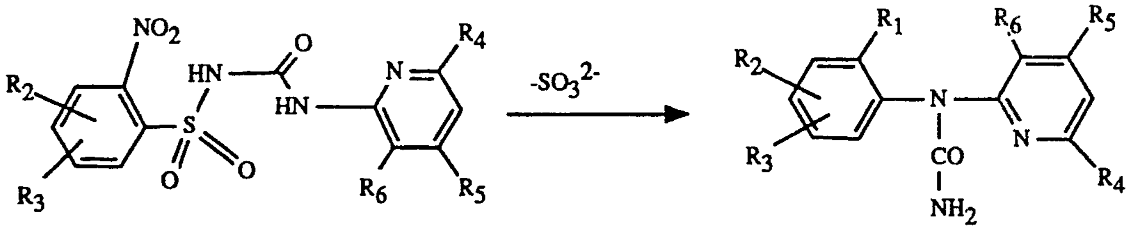


(VI) (I') ($R^1 = NO_2$)

13. Verfahren zur Herstellung von Anilinen der Formel II, worin die Reste $R_1$ bis $R_6$ wie in Anspruch 1 unter Formel I definiert sind, dadurch gekennzeichnet, dass man

a) ein Anilin der Formel VII, worin die Reste $R_1$ bis $R_3$ wie zuvor definiert sind, mit einem Pyridin der Formel III, worin die Reste $R_4$ bis $R_6$ wie unter Formel II definiert sind und X Halogen, $C_1$-$C_4$-Alkyl-$SO_2$-, oder Phenyl-$SO_2$- bedeutet, unter Baseneinwirkung umsetzt

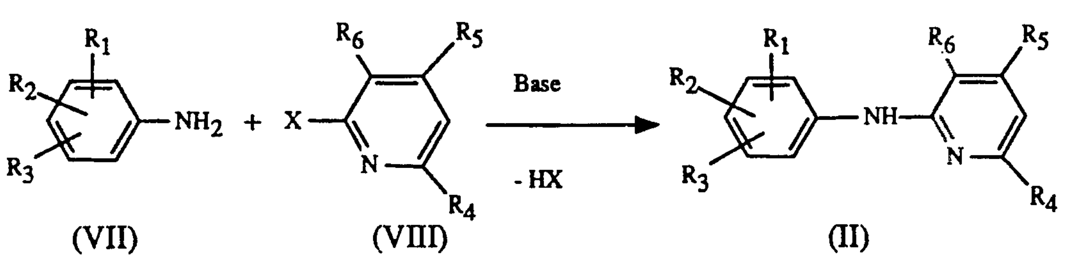


(VII) (VIII) (II)

oder

b) ein Halogenbenzol der Formel IX, worin die Reste $R_1$, $R_2$ und $R_3$ wie unter Formel II definiert sind und Y Halogen bedeutet, unter Baseneinwirkung mit einem 2-Aminopyridin der Formel X umsetzt

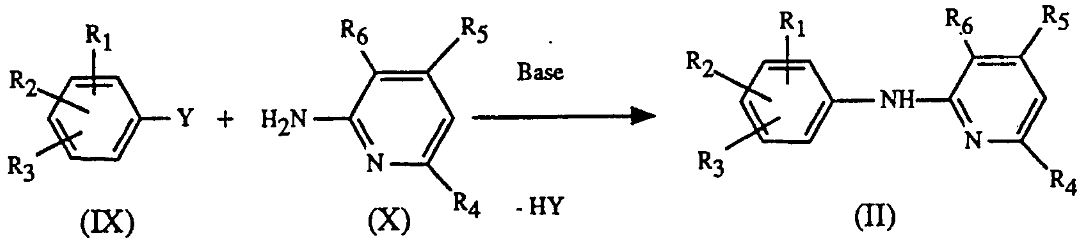


(IX) (X) (II)

14. Verfahren zur Herstellung von Verbindungen der Formel III worin die Reste $R_1$ bis $R_6$ wie in Anspruch 1 unter Formel I definiert sind, dadurch gekennzeichnet, dass man ein Anilin der Formel II mit Phosgen umsetzt

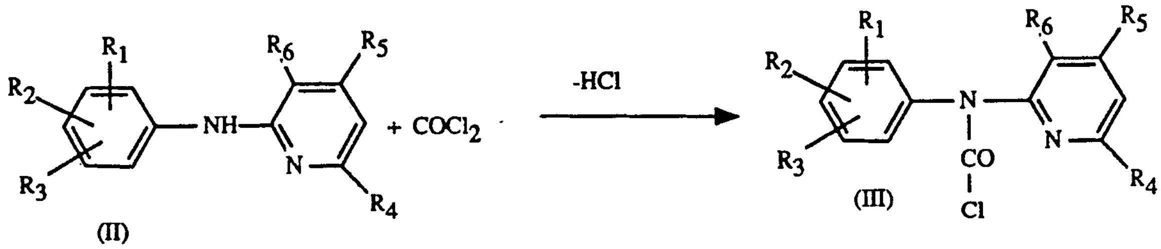


(II) (III)

15. Verfahren zur Herstellung von Verbindungen der Formel IV,

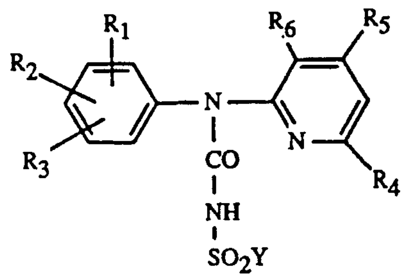

(IV),

worin die Reste $R_1$ bis $R_6$ wie in Anspruch 1 unter Formel I definiert sind, dadurch gekennzeichnet, dass man ein Anilin der Formel II, worin die Reste $R_1$ bis $R_6$ wie unter Formel IV definiert sind, mit einem Halogensulfonylisocyanat der Formel V, worin Y für Halogen steht umsetzt

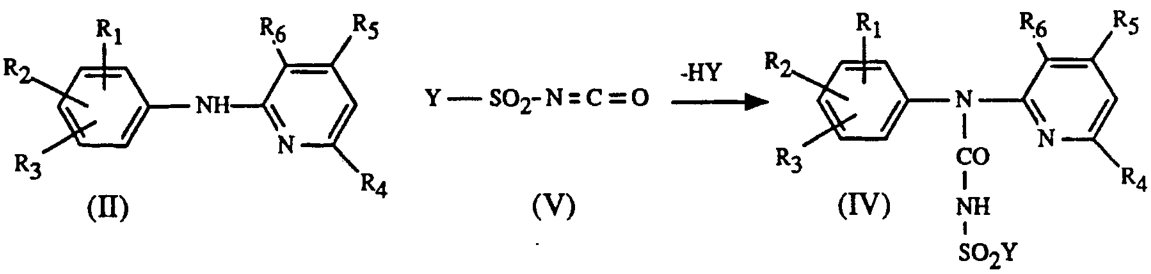


(II) (V) (IV)

16. Verfahren zur Bekämpfung unerwünschten Pflanzenwuchses, dadurch gekennzeichnet, dass man eine herbizid wirksame Menge eines Mittels gemäss Anspruch 1 auf die zu bekämpfende Pflanze oder deren Lebensraum einwirken lässt.

17. Verfahren gemäss Anspruch 16 zur pre- oder postemergenten Bekämpfung unerwünschten Pflanzenwuchses in Nutzpflanzenkulturen.

18. Verfahren zur Beeinflussung des Pflanzenwuchses, dadurch gekennzeichnet, dass man eine pflanzenwuchsregulatorisch wirksame Menge eines Mittels gemäss Anspruch 1 auf die Pflanze oder deren Lebensraum einwirken lässt.

19. Saatgut, welches mit einer herbizid oder pflanzenwuchsregulatorisch wirksamen Menge eines Mittels gemäss Anspruch 1 behandelt worden ist.